# EUROPEAN PATENT APPLICATION

(11) **EP 4 431 150 A2**
(43) Date of publication of application: **18.09.2024**
(21) Application number: 24180561.3
(22) Date of filing: 08.12.2022
(51) Int. Cl.: A61P 11/00

(54) **MUC5B-TARGETED ANTISENSE OLIGONUCLEOTIDES AND RELATED METHODS FOR MODULATING MUCIN EXPRESSION**

(30) Priority: 09.12.2021 US 202163287554 P; 30.01.2022 US 202263304622 P
(62) Divisional of application: 22903735.3
(71) Applicant: Splisense Ltd., 9139002 Jerusalem (IL)
(72) Inventor: HART, Gili, 6085001 Shoham (IL); OZERI-GALAI, Efrat, 9626431 Jerusalem (IL); OREN, Yifat, 9618229 Jerusalem (IL); BARCHAD-AVITZUR, Ofra, 9338519 Jerusalem (IL); STAMPFER, Chava, 9045701 Efrat (IL)
(74) Representative: Pearl Cohen Zedek Latzer Baratz UK LLP

(57) **Abstract**

The present invention provides specific synthetic oligonucleotides targeting MUC5B, as well as vectors, cells, and pharmaceutical compositions comprising the oligonucleotides, and their use in treating, suppressing, inhibiting, ameliorating, or slowing progression of a lung disease or disorder, such as chronic obstructive pulmonary disease (COPD), asthma, idiopathic pulmonary fibrosis (IPF), and non-cystic fibrosis bronchiectasis (NCFB).

## Description

### SEQUENCE LISTING STATEMENT

A Sequence Listing conforming to the rules of WIPO Standard ST.26 is hereby incorporated by reference. The Sequence Listing has been filed as an electronic document via EFS-Web in ASCII format encoded as XML. The electronic document, created on December 7, 2022, is entitled "P-610294-PC_ST26.xml", and is 3,869,470 bytes in size.

### FIELD OF THE DISCLOSURE

The present invention provides specific synthetic oligonucleotides, as well as vectors, cells, and pharmaceutical compositions comprising the oligonucleotides, and their use in methods of treating, suppressing, inhibiting, ameliorating, or slowing progression of a lung disease or disorder, such as chronic obstructive pulmonary disease (COPD), asthma, idiopathic pulmonary fibrosis (IPF), and non-cystic fibrosis bronchiectasis (NCFB).

### BACKGROUND

Mucus is a thin extracellular hydrogel film consisting of water, ions, proteins, and macromolecules covering airway surfaces that is involved in the protection, lubrication, and transport through airways. Under healthy conditions, mucus maintains hydration in the airways and protects against inhaled pathogens, toxic gases, and airborne pollutants. Mucus also helps the innate immune response by transporting a variety of antioxidants, antiproteases, antimicrobial substances, immune-modulatory molecules, and protective molecules. However, excessive mucus contributes to transient respiratory infections and to the pathogenesis of numerous respiratory diseases.

The major macromolecular components of mucus are the mucin glycoproteins, which are critical for local defense of the airway. Healthy airway mucus is comprised mainly of water (>95%). The primary solid components of the mucus layer are the mucins, which are produced by epithelial cells and mucin-secreting goblet cells and give mucus its viscoelastic properties. Mucins are high molecular-weight glycoproteins (~3 MDa), 50-90% carbohydrate by mass and with dozens to several hundreds of O-glycosylation sites per molecule.

Increased mucin production occurs in many adenocarcinomas, including cancers of the pancreas, lung, breast, ovary, colon and other tissues. Mucins are also overexpressed in lung diseases such as asthma, bronchitis, chronic obstructive pulmonary disease (COPD), idiopathic pulmonary fibrosis, and cystic fibrosis.

Muco-obstructive airway diseases are characterized by mucin-producing cell hyperplasia, mucin hypersecretion, and an altered mucin macromolecular form, which contribute to the formation of a dysfunctional mucus gel. Mucus accumulated in the airways cause obstruction which results in infection and inflammation.

Also, mucus with aberrant properties compromises airway clearance. The mucus may be characterized by defective mucin intracellular assembly and intragranular packaging, defective post-secretory mucin expansion as a result of a non-optimal local environment (hydration, pH and HCO₃), formation of permanent cross-links between mucin chains, alterations in the relative amounts of MUC5AC and MUC5B, and/or alterations in the different glycosylated variants of MUC5B.

Antisense oligonucleotides (ASOs) are small synthetic nucleic acid molecules able to bind specific sequences within target RNA molecules. ASOs are used for gene silencing by RNA cleavage and RNA editing. ASOs may also be used to modulate the splicing of a target gene by enhancing retention or skipping of a specific exon. Chemical modifications of ASOs allow ASOs to align with the target sequence, such as a consensus motif, without inducing cleavage of the target RNA. ASOs are highly specific and allow manipulation of specific exons without influencing the splicing of other genes.

Using ASOs to specifically target splicing sites in mucin mRNA rather than for gene silencing is a novel way for controlling expression or overexpression of mucin mRNA and provides a much-needed alternative treatment for muco-obstructive disorders and cancer.

### SUMMARY OF THE DISCLOSURE

In one embodiment, the present invention provides a synthetic antisense oligonucleotide targeting MUC5AC, MUC5B, or both MUC5AC and MUC5B sequences, wherein said oligonucleotide is fully chemically modified.

In another embodiment, the present invention provides a synthetic antisense oligonucleotide which targets exons 4-8, 10-11, 13, 16-22, 26, 28-29, or 31 of MUC5AC, MUC5B, or both MUC5AC and MUC5B.

In another embodiment, the present invention provides a vector comprising a synthetic antisense oligonucleotide targeting MUC5AC, MUC5B, or both MUC5AC and MUC5B sequences, as described herein.

In another embodiment, the present invention provides a pharmaceutical composition comprising a synthetic antisense oligonucleotide targeting MUC5AC, MUC5B, or both MUC5AC and MUC5B sequences, as described herein.

In another embodiment, the present invention provides a method for treating a lung disease or disorder in a subject, comprising the step of administering to said subject a synthetic antisense oligonucleotide targeting MUC5AC, MUC5B, or both MUC5AC and MUC5B sequences, as described herein, or a vector, cell, or pharmaceutical composition comprising the antisense oligonucleotide.

In another embodiment, the present invention provides a method for suppressing or inhibiting a lung disease or disorder in a subject, comprising the step of administering to said subject a synthetic antisense oligonucleotide targeting MUC5AC, MUC5B, or both MUC5AC and MUC5B sequences, as described herein, or a vector, cell, or pharmaceutical composition comprising the antisense oligonucleotide.

In another embodiment, the present invention provides a method for reducing the levels of MUC5AC, MUC5B, or both MUC5AC and MUC5B in a cell of a subject comprising the step of administering to said subject a synthetic antisense oligonucleotide targeting MUC5AC, MUC5B, or both MUC5AC and MUC5B sequences, as described herein, or a vector, cell, or pharmaceutical composition comprising the antisense oligonucleotide.

In another embodiment, the present invention provides a method of inducing nonsense-mediated decay of MUC5AC, MUC5B, or both MUC5AC and MUC5B in a cell of a subject, comprising the step of administering to the subject a composition comprising a synthetic antisense oligonucleotide targeting MUC5AC, MUC5B, or both MUC5AC and MUC5B sequences as described herein.

### BRIEF DESCRIPTION OF THE DRAWINGS

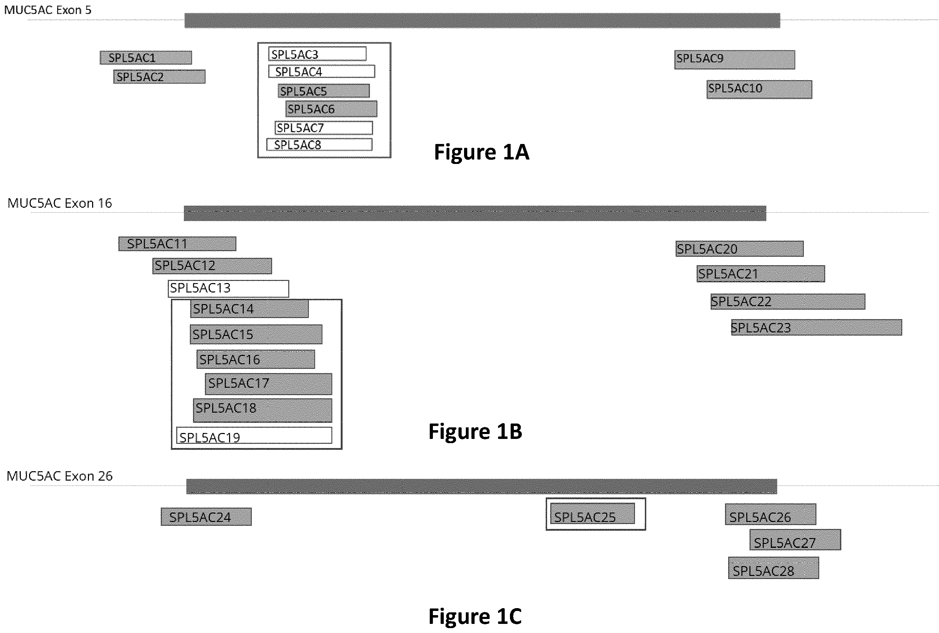

**Figure 1****. A map of the MUC5AC ASOs tested.** The tested human ASOs are mapped to their positions relative to exon 5 **(****Figure 1A****),** exon 16 **(****Figure 1B****)** or exon 26 **(****Figure 1C****)** and flanking intronic sequences (thin gray bar). Boxes surround ASOs located in areas that are 100% homologous to the mouse Muc5ac gene. ASOs having the strongest effect on lowering MUC5AC RNA levels have a white background.

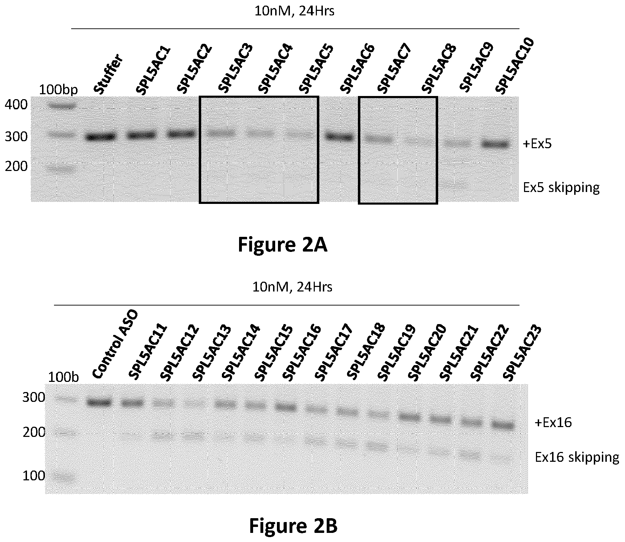

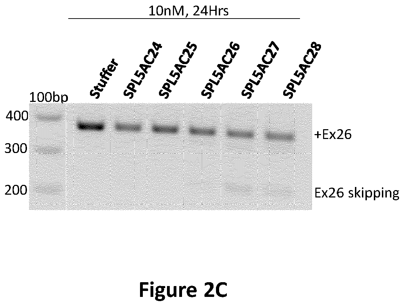

**Figure 2****. The effect of MUC5AC ASOs on the level of MUC5AC transcript.** A549 cells were transfected with 10nM of each ASO, RNA was extracted, and the levels of MUC5AC transcripts were analyzed by RT-PCR 24 hours after transfection. **Figure 2A****.** An example of RT-PCR of ASOs screened for exon 5. **Figure 2B****.** An example of RT-PCR of ASOs screened for exon 16. **Figure 2C****.** An example of RT-PCR of ASOs screened for exon 26. Boxed lanes indicate the ASOs that are most efficient in reducing full length MUC5AC RNA.

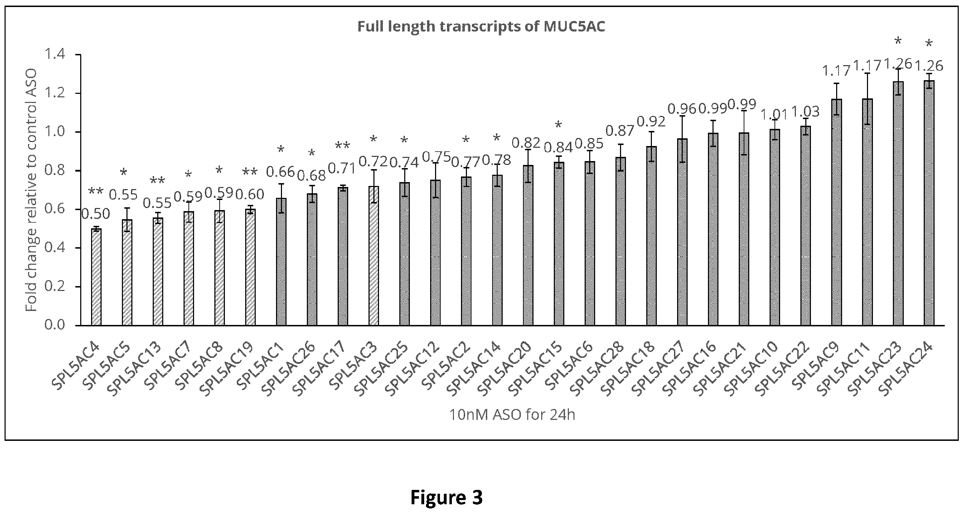

**Figure 3****. MUC5AC full length transcript levels following ASO transfection.** Following a 24-hour treatment of A549 cells with 10nM of the indicated ASO, RNA was extracted and the level of MUC5AC full-length transcript was measured by RT-qPCR 24 hours after transfection. The values shown are the average fold change (mean±SEM) from 3-4 independent experiments relative to cells treated with a control ASO. Values were normalized against transcripts of GAPDH gene. Bars representing ASOs that reduced the level of full length MUC5AC transcripts to 60% of control levels or less are striped, as well as SPL5AC3, which showed an effect in RT-PCR. Bars representing ASOs that are less efficient are gray. Statistical analysis was performed using Student's t test (1 tail, paired). *p<0.05, **p<0.01.

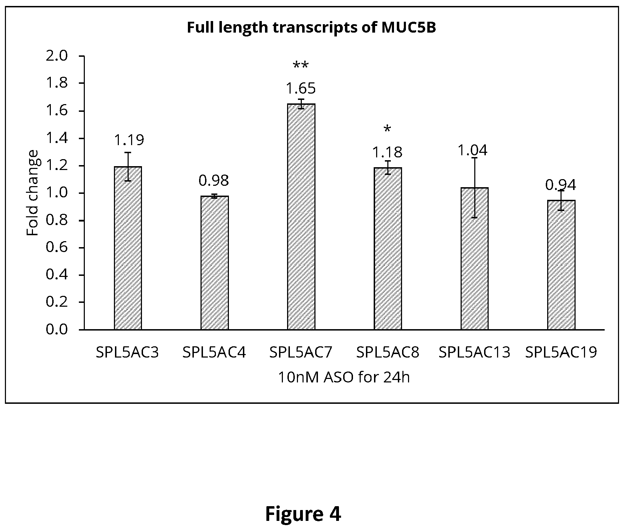

**Figure 4****. Off-target effect of selected MUC5AC-targeted ASOs on the MUC5B full length transcripts.** MUC5B full length transcripts levels in A549 cells following ASO transfection (10nM). Following a 24-hour treatment with the indicated ASO, RNA was extracted and the levels of MUC5B full-length transcripts were measured by RT-qPCR. The values shown are the average fold change (mean±SEM) from 3-4 independent experiments relative to cells treated with a control ASO. Values were normalized against transcripts of GAPDH gene. Statistical analysis was performed using Student's t-test (1 tail, paired). *p<0.05, **p<0.01.

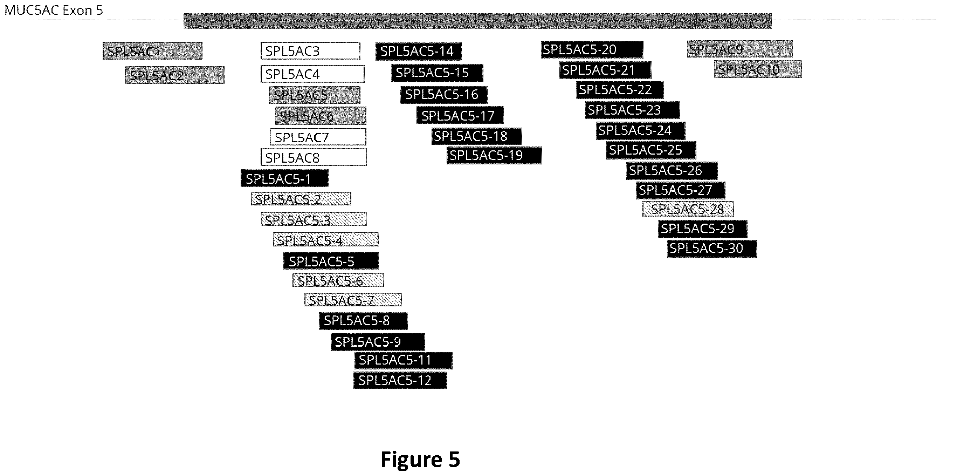

**Figure 5****. A map of ASOs targeting Exon 5 of MUC5AC.** The ASOs are mapped to their position relative to exon 5.

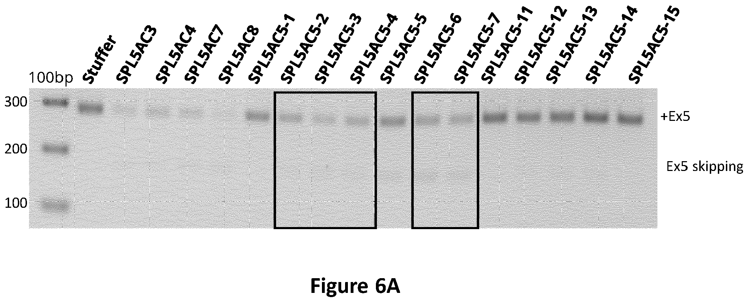

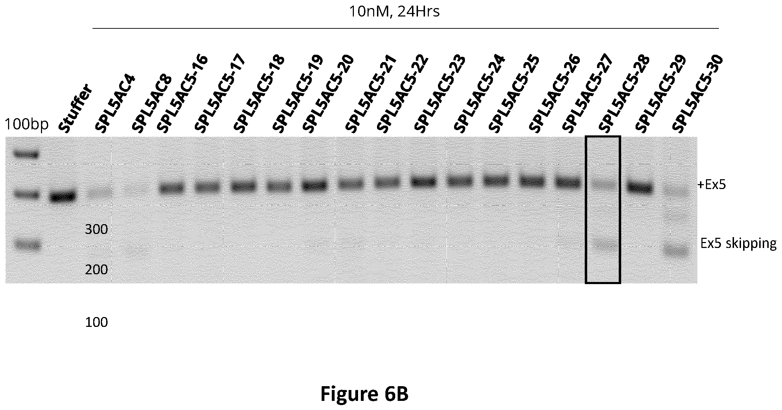

**Figure 6****. The effect of the MUC5AC Exon 5 ASOs on MUC5AC transcript levels detected by RT-PCR.** A549 cells were transfected with 10nM of each ASO, RNA was extracted, and the levels of MUC5AC transcripts were analyzed 24 hours after transfection. **Figure 6A****.** An example of RT-PCR of ASOs SPL5AC5-1 through SPL5AC5-15. **Figure 6B****.** An example of RT-PCR of ASOs SPL5AC5-16 through SPL5AC5-30. Boxed lanes indicate the ASOs that are most efficient in reducing full length MUC5AC RNA

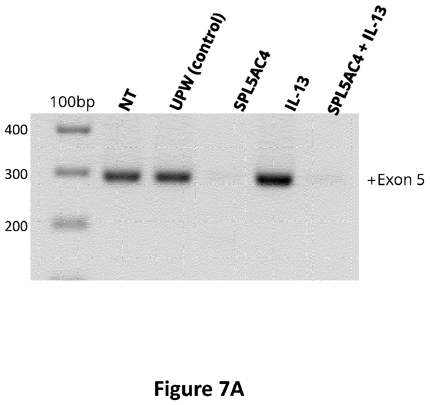

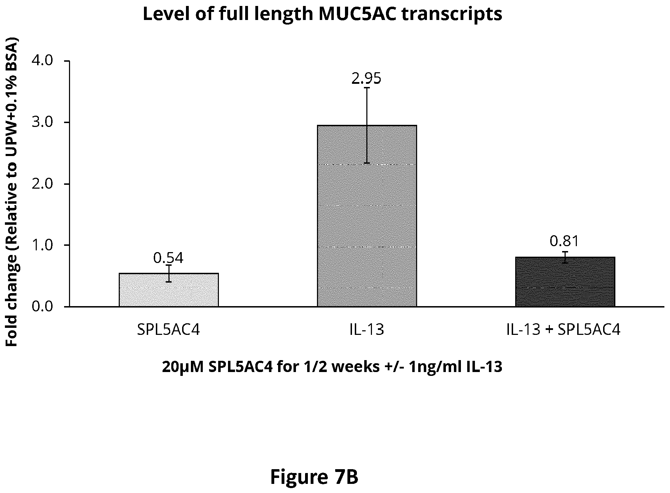

**Figure 7****. The effect of the MUC5AC Exon 5-directed SPLAC4 ASO on the MUC5AC transcripts level in human nasal epithelial (HNE) cells.** HNEs were differentiated in air-liquid conditions for 35 days. Cells were treated with 20µM SPL5AC4 every other day for the last two weeks of differentiation prior to RNA harvest, for a total of 7 additions. Some cells were treated with 1ng/ml IL-13, which stimulates mucus production and induces MUC5AC expression in human airway epithelial cells, two days prior to adding the ASO and every time the ASO was added (8 additions in total). On day 35 of differentiation, RNA was extracted and the levels of MUC5AC full-length transcripts were measured by RT-PCR **(****Figure 7A**) and RT-qPCR **(****Figure 7B**). The values shown are the average fold change (mean±SEM) from 3-4 filters relative to cells treated with UPW+ 0.1% BSA (control). Values were normalized against transcripts of GUSb gene.

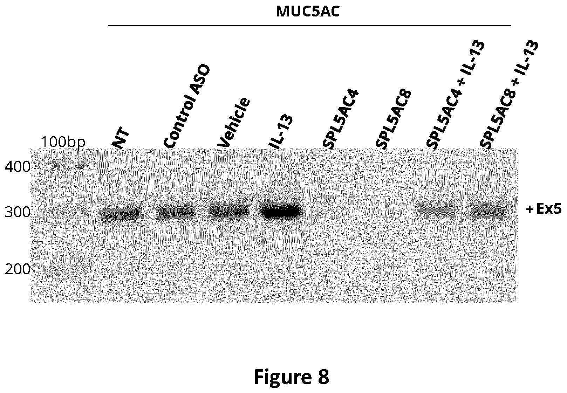

**Figure 8****. The effect of MUC5AC Exon 5-directed SPLAC4 ASO and SPLAC8 ASO on the level of MUC5AC transcripts in human bronchial epithelial (HBE) cells.** HBEs were differentiated in air-liquid conditions for 35 days. Cells were treated with 20µM SPL5AC4 or SPL5AC8 for the last two weeks of differentiation every other day prior to RNA harvest, for a total of 7 additions. Some cells were treated with 10ng/ml IL-13, which stimulates mucus production and induces MUC5AC expression in human airway epithelial cells, two days prior to adding the ASO and every time the ASO was added (8 additions in total). On day 35 of differentiation, RNA was extracted and the levels of MUC5AC full-length transcripts were measured by RT-PCR.

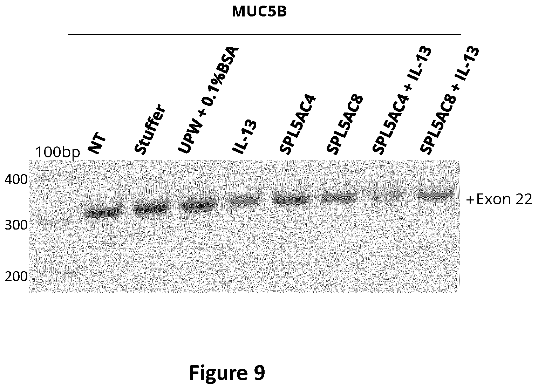

**Figure 9****. No off-target effect of SPLAC4 on the splicing pattern of the MUC5B gene in HBE cells.** HBEs were differentiated in air-liquid conditions for 35 days. Cells were treated with 20µM SPL5AC4 or SPL5AC8 every other day for the last two weeks of differentiation prior to RNA harvest, for a total of 7 additions. Some cells were treated with 1ng/ml IL-13, which stimulates mucus production and induces MUC5AC expression in human airway epithelial cells, two days prior to adding the ASO and every time the ASO was added (8 additions in total). On day 35 of differentiation, RNA was extracted and the levels of MUC5B full-length transcripts were measured by RT-PCR.

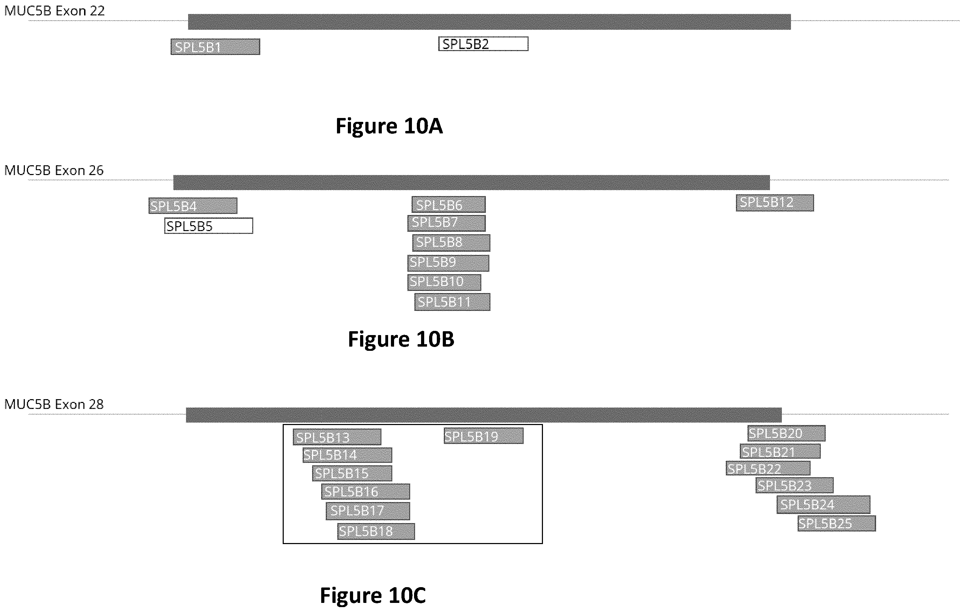

**Figure 10****: A map of the MUC5B ASOs tested and their origin ASOs.** The tested human ASOs are mapped to their positions relative to exon 22 **(****Figure 10A****),** exon 26 **(****Figure 10B****)** or exon 28 **(****Figure 10C****)** and flanking intronic sequences (thin gray bar). Boxes surround ASOs located in areas that are 100% homologous to the mouse Muc5b gene. ASOs having the strongest effect on lowering MUC5AC RNA levels have a white background.

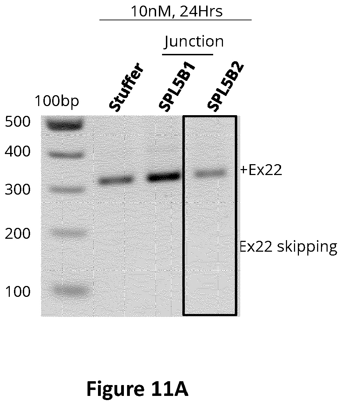

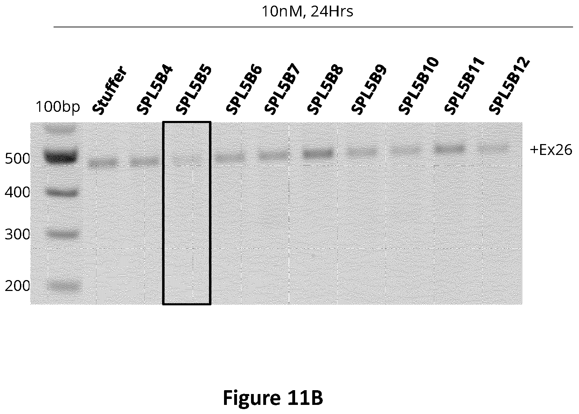

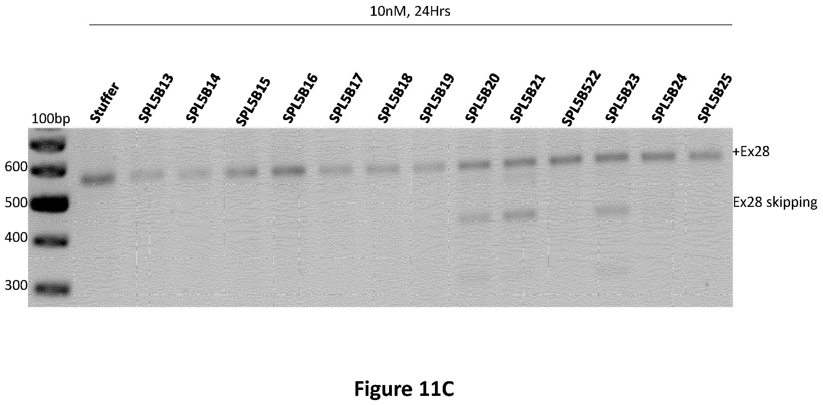

**Figure 11****. The effect of MUC5B ASOs on the level of MUC5B transcript.** A549 cells were transfected with 10nM of each ASO, RNA was extracted, and the levels of MUC5B transcripts were analyzed by RT-PCR 24 hours after transfection. **Figure 11A****.** An example of RT-PCR of ASOs screened for exon 22. **Figure 11B****.** An example of RT-PCR of ASOs screened for exon 26. **Figure 11C****.** An example of RT-PCR of ASOs screened for exon 28.

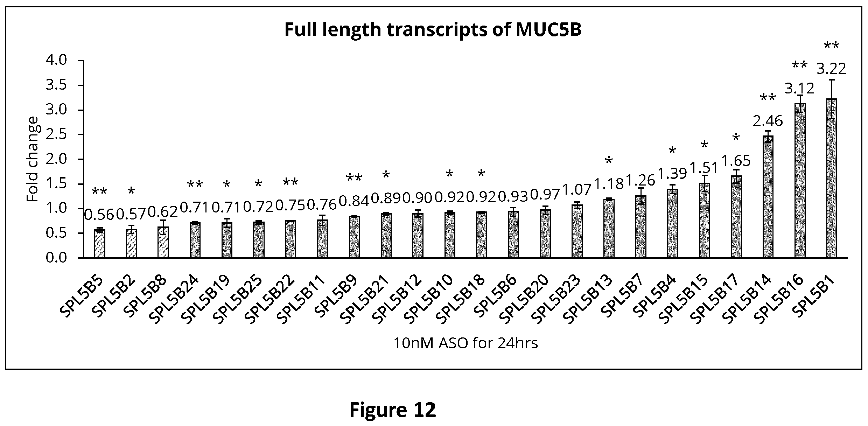

**Figure 12****. MUC5B full length transcript levels in A549 cells following ASO transfection.** Following a 24-hour treatment with 10nM of the indicated ASO, RNA was extracted, and the levels of MUC5B full-length transcripts were measured by RT-qPCR 24 hours after transfection. The values shown are the average fold change (mean±SEM) from 3-4 independent experiments relative to cells treated with a control ASO. Values were normalized against transcripts of GAPDH gene. Bars representing ASOs that reduced the level of full length MUC5B transcripts to 65% of control levels or less are striped, while bars representing ASOs that are less efficient are gray. Statistical analysis was performed using Student's t test (1 tail, paired). *p<0.05, **p<0.01.

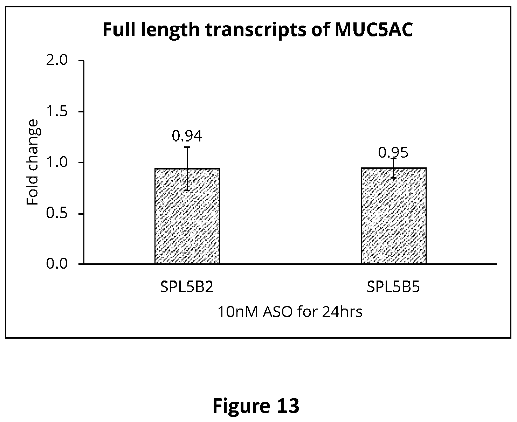

**Figure 13****. Off-target effect of the selected MUC5B-targeted ASOs on the MUC5AC full length transcripts.** MUC5AC full length transcripts levels in A549 cells following ASO transfection (10nM). Following a 24-hour treatment with the indicated ASO, RNA was extracted and the levels of MUC5AC full-length transcripts were measured by RT-qPCR. The values shown are the average fold change (mean±SEM) from 3-4 independent experiments relative to cells treated with a control ASO. Values were normalized against transcripts of GAPDH gene. Statistical analysis was performed using Student's t test (1 tail, paired).

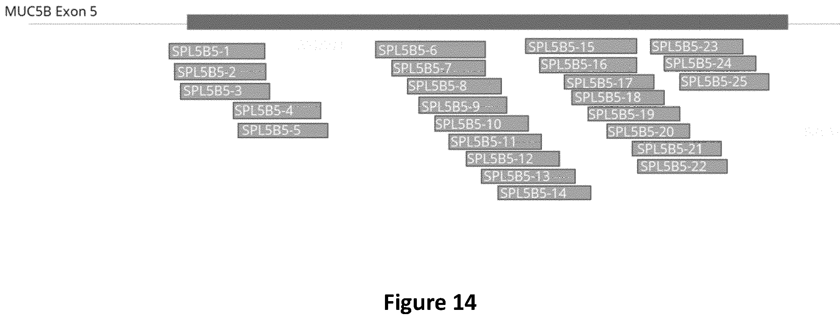

**Figure 14****. A map of ASOs tested that target Exon 5 of MUC5B.** The ASOs are mapped to their position relative to exon 5.

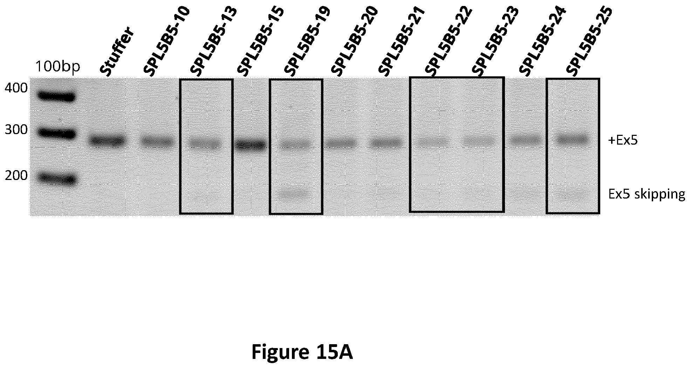

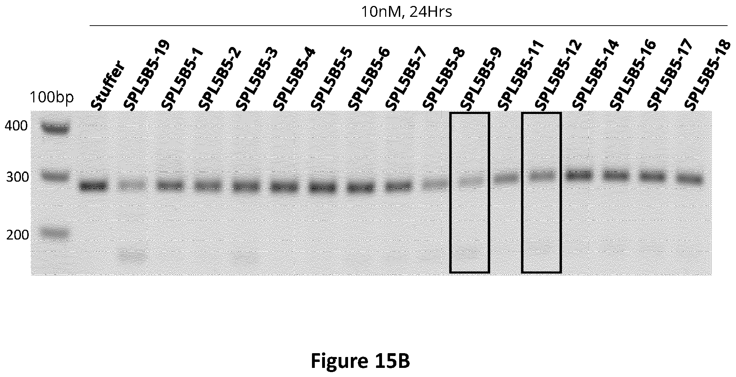

**Figure 15****. The effect of the MUC5B Exon 5 ASOs on MUC5B transcript levels detected by RT-PCR.** A549 cells were transfected with 10nM of each ASO, RNA was extracted, and the levels of MUC5B transcripts were analyzed 24 hours after transfection. **Figure 15A****.** An example of RT-PCR of ASOs SPL5B5-10, SPL5B5-13, SPL5B5-15, SPL5B5-19, and SPL5B5-21 through SPL5B5-25. **Figure 15B****.** An example of RT-PCR of ASOs SPL5B5-1- SPL5B5-9, SPL5B5-11, SPL5B5-12, SPL5B5-14, and SPL5B5-16 through SPL5B5-19. Boxed lanes indicate the ASOs that are most efficient in reducing full length MUC5B RNA

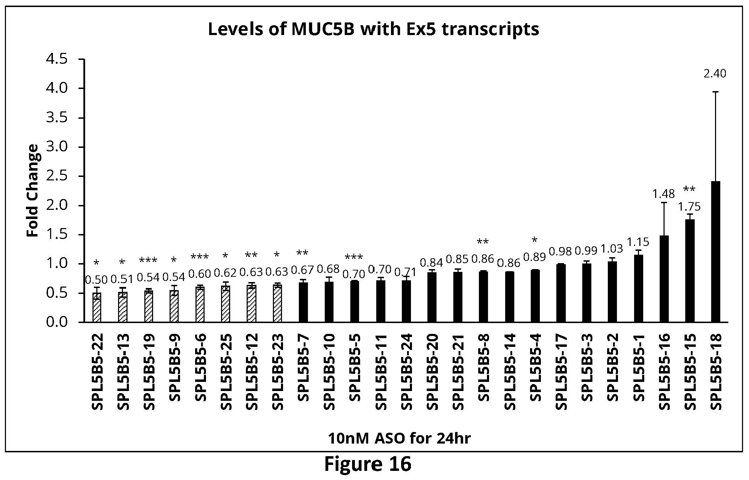

**Figure 16****. MUC5B full length transcript levels following transfection with Exon 5-targeting ASOs.** Following a 24-hour treatment of A549 cells with 10nM of the indicated ASO, RNA was extracted and the level of full length MUC5B transcripts was measured by RT-qPCR 24 hours after transfection. The values shown are the average fold change (mean±SEM) from 3-4 independent experiments relative to cells treated with a control ASO. Values were normalized against transcripts of GAPDH gene. Bars representing ASOs that reduced the level of full length MUC5B transcripts to 65% of control levels or less are striped, while bars representing ASOs that are less efficient are black. Statistical analysis was performed using Student's t-test (1 tail, paired). *p<0.05, **p<0.01, ***p<0.001.

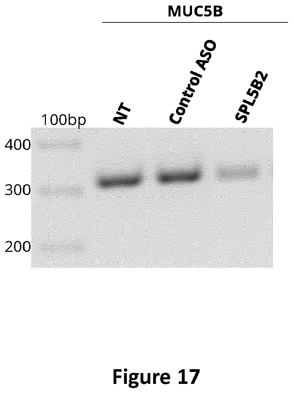

**Figure 17****. The effect of MUC5B Exon 5-directed SPL5B2 ASO on the level of MUC5B transcripts in human bronchial epithelial (HBE) cells.** HBEs were differentiated in air-liquid conditions for 35 days. Cells were treated with 20µM SPL5B2 every other day for the last two weeks of differentiation prior to RNA harvest for a total of 7 additions. On day 35 from differentiation, RNA was extracted and the levels of MUC5B full-length transcripts were measured by RT-PCR.

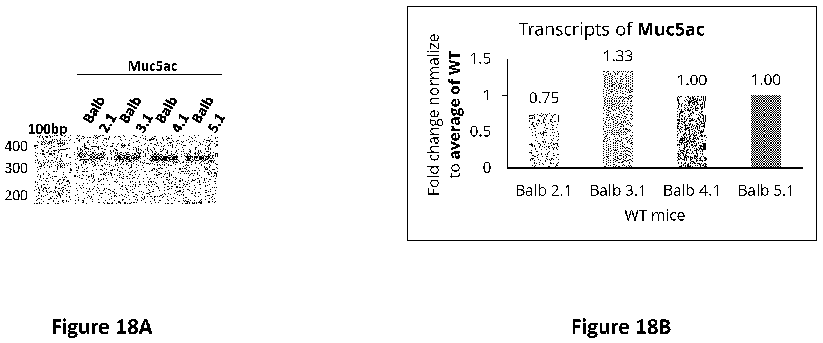

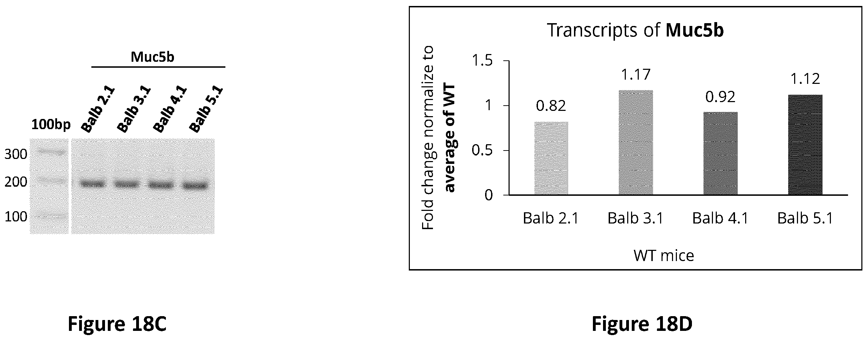

**Figure 18****. Baseline Muc5ac and Muc5b RNA expression levels in mice.** RNA was extracted from the lungs of 4 Balb/c mice, and the levels of Muc5ac and Muc5b transcripts were detected by RT-PCR **(****Figure 18A** and **Figure 18C****)** and RT-qPCR **(****Figure 18B** and **Figure 18D****).** The values shown are the fold change relative to the average of the values for WT mice. Values were normalized against transcripts of IPO8 gene.

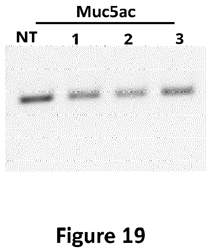

**Figure 19****. SPL5AC4 reduces Muc5ac levels in Balb/c mice.** Balb/c mice were administered **SPL5AC4** intrathecally (IT) 4 times over two weeks at a dose of 10 mg/kg/dose. RNA was extracted from the lungs, and the levels of Muc5ac transcripts were detected by RT-PCR.

### DETAILED DESCRIPTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The present invention provides compositions and methods for treating a lung disease or disorder in a subject by administering antisense oligonucleotides that alter the splicing of MUC5AC, MUC5B, or both MUC5AC and MUC5B gene transcripts. In some embodiments, administration of the antisense oligonucleotides as described herein reduces MUC5AC, MUC5B, or both MUC5AC and MUC5B mRNA levels. In some embodiments, administration of the antisense oligonucleotides as described herein reduces MUC5AC, MUC5B, or both MUC5AC and MUC5B protein expression. In some embodiments, administration of the antisense oligonucleotides as described herein introduces a premature termination codon (PTC) in the MUC5AC, MUC5B, or both MUC5AC and MUC5B gene transcripts. In some embodiments, the truncated MUC5AC, MUC5B, or both MUC5AC and MUC5B gene transcripts are degraded by the Nonsense-Mediated Decay (NMD) mechanism. In some embodiments, administration of the antisense oligonucleotides as described herein increases mucin-specific mRNA degradation. In other embodiments, administration of the antisense oligonucleotides as described herein decreases expression levels, cellular localization or function of MUC5AC, MUC5B, or both MUC5AC and MUC5B, or a combination thereof. In some embodiments, the compositions and methods as described herein inhibit expression of MUC5AC, MUC5B, or both MUC5AC and MUC5B mRNA and/or protein, but do not eliminate expression of MUC5AC, MUC5B, or both MUC5AC and MUC5B mRNA and/or protein. In some embodiments, altered expression or activity of MUC5AC, MUC5B, or both MUC5AC and MUC5B is in airway cells. In other embodiments, expression of activity is in mucus secreting airway cells.

### Antisense Oligonucleotides

In some embodiments, "antisense oligonucleotide" or "ASO" as used herein describes a single-stranded oligonucleotide having a nucleobase sequence that permits hybridization to a corresponding segment of a target nucleic acid.

In some embodiments, the present invention provides an oligonucleotide, which is a splicing modulator. In some embodiments, the present invention provides vectors, cells, compositions, methods, and kits as described herein comprise a splicing modulator. In some embodiments, the present invention provides administration of a splicing modulator, which, in some embodiments, is an antisense oligonucleotide.

In some embodiments, the present invention provides an antisense oligonucleotide that targets a MUC pre-mRNA. In some embodiments, the MUC pre-mRNA comprises MUC2, MUC19, MUC1, MUC4, MUC7, MUC11, MUC15, MUC16, MUC20, or a combination thereof. In other embodiments, the MUC pre-mRNA comprises MUC5 pre-mRNA. In some embodiments, the MUC5 pre-mRNA comprises MUC5AC. In other embodiments, the MUC5 pre-mRNA comprises MUC5B. In some embodiments, the ASO targets a mucin that is secreted, which in one embodiment, comprises MUC7, MUC5AC, MUC5B. In some embodiments, the ASO targets a mucin that is cell surface associated, which in one embodiment, comprises MUC1, MUC4, MUC16, MUC20. In some embodiments, the ASO targets a mucin that polymerizes to form gels, while in other embodiments, the ASO targets a mucin that does not polymerize, which in one embodiment is MUC7.

In some embodiments, the present invention provides an oligonucleotide having 8 to 30 linked nucleosides having a nucleobase sequence comprising a complementary region, wherein the complementary region comprises at least 8 contiguous nucleobases complementary to an equal-length portion of a target region of a MUC5AC, MUC5B, or both MUC5AC and MUC5B transcript. In other embodiments, the antisense oligonucleotide has a nucleobase sequence comprising at least 12, at least 13, at least 14, at least 15, at least 16, at least 17, at least 18, or at least 19 contiguous nucleobases.

In some embodiments, targeting includes determination of at least one target segment to which an oligonucleotide hybridizes, such that a desired effect occurs. A target region may contain one or more target segments. Multiple target segments within a target region may be overlapping. Alternatively, they may be non-overlapping. In some embodiments, target segments within a target region are separated by no more than about 300 nucleotides. In some embodiments, target segments within a target region are separated by a number of nucleotides that is no more than 250, 200, 150, 100, 90, 80, 70, 60, 50, 40, 30, 20, or 10 nucleotides on the target nucleic acid, or is a range defined by any two of the preceding values. In some embodiments, target segments within a target region are separated by no more than five nucleotides on the target nucleic acid. In some embodiments, target segments are contiguous.

In some embodiments, a target region is a structurally defined region of the target nucleic acid. In some embodiments, a suitable target segment may be found within a 5' UTR, a coding region, a 3' UTR, an intron, an exon, or an exon/intron junction. In one embodiment, the target segment is in an exon/intron junction. In some embodiments, the target segment is within an exon of MUC5AC or MUC5B. In some embodiments, the target segment is an exonic splicing silencer. In other embodiments, the target segment is an exonic splicing enhancer. In other embodiments, the target region comprises a translation initiation region, translation termination region, or other defined nucleic acid region. The structurally defined regions for MUC5A or MUC5B can be obtained by accession number from sequence databases such as NCBI and such information is incorporated herein by reference.

In some embodiments, the antisense oligonucleotide is complementary to and/or targeted to MUC5AC (for example, NCBI accession numbers: NM_001304359.2, AF015521.1, AF043909.1, AJ001402.1, AJ292079.1, AJ298317.1, AL833060.1, BC033831.1, L46721.1, U06711.1, X81649.1, Z34277.1, Z34278.1, Z34279.1, Z34280.1, Z34281.1, Z34282.1, Z34283.1, Z48314.1). In other embodiments, the antisense oligonucleotide is complementary to and/or targeted to MUC5B (for example, NCBI accession numbers: NM_002458.3, AA577624.1, AF086604.1, AF253321.1, AK303892.1, AK310939.1, BM740816.1, BM796192.1, BM817984.1, CA450235.1, DC418119.1, S80993.1, U63836.1, U78550.1, U78551.1, U95031.1, X74370.1, X74954.1, X74955.1, X74956.1). In other embodiments, the antisense oligonucleotide is complementary to and/or targeted to a portion of MUC5AC or to a portion of MUC5B. In other embodiments, the antisense oligonucleotide is complementary to and/or targeted to both MUC5AC and MUC5B pre-mRNA.

In some embodiments, the nucleobases of the antisense oligonucleotide as described herein are complementary to a portion of SEQ ID NO: 2306. In another embodiment, the nucleobases of the antisense oligonucleotide as described herein are complementary to a portion of SEQ ID NO: 2307.

In some embodiments, the nucleobases of the antisense oligonucleotide are complementary to one or more portions of exons 1-31 of MUC5AC, MUC5B, or both MUC5AC and MUC5B. In other embodiments, the nucleobases of the antisense oligonucleotide are complementary to one or more portions of exons 4-29 of MUC5AC, MUC5B, or both MUC5AC and MUC5B. In other embodiments, the nucleobases of the antisense oligonucleotide are complementary to one or more portions of exons 4-8 of MUC5AC, MUC5B, or both MUC5AC and MUC5B. In other embodiments, the nucleobases of the antisense oligonucleotide are complementary to one or more portions of exons 10-11 of MUC5AC, MUC5B, or both MUC5AC and MUC5B. In other embodiments, the nucleobases of the antisense oligonucleotide are complementary to one or more portions of exon 13 of MUC5AC, MUC5B, or both MUC5AC and MUC5B. In other embodiments, the nucleobases of the antisense oligonucleotide are complementary to one or more portions of exons 16-22 of MUC5AC, MUC5B, or both MUC5AC and MUC5B. In other embodiments, the nucleobases of the antisense oligonucleotide are complementary to one or more portions of exon 26 of MUC5AC, MUC5B, or both MUC5AC and MUC5B. In other embodiments, the nucleobases of the antisense oligonucleotide are complementary to one or more portions of exons 28-29 of MUC5AC, MUC5B, or both MUC5AC and MUC5B. In other embodiments, the nucleobases of the antisense oligonucleotide are complementary to one or more portions of exon 31 of MUC5AC, MUC5B, or both MUC5AC and MUC5B.

In other embodiments, the nucleobases of the antisense oligonucleotide are complementary to one or more portions of Exons 4-8, 10-11, 13, 16-22, 26, 28-29, or 31 of MUC5AC, MUC5B, or both MUC5AC and MUC5B. In other embodiments, the ASO targets exons 4-8, 10-11, 13, 16-22, 26, 28-29, or 31 of MUC5AC, MUC5B, or both MUC5AC and MUC5B and/or introns adjacent to said exons. In other embodiments, the ASO targets exon 5, exon 16, or exon 26 of MUC5AC. In other embodiments, the ASO targets a portion of exon 5, exon 16, or exon 26 of MUC5AC. In other embodiments, the ASO targets exon 5, exon 22, exon 26, or exon 28 of MUC5B. In other embodiments, the ASO targets a portion of exon 5, exon 22, exon 26, or exon 28 of MUC5B.

In some embodiments, the nucleobases of the antisense oligonucleotide as described herein are complementary to a portion of any one or more of SEQ ID NOs: 215-232 or 272 or SEQ ID NOs: 2290-2297 or SEQ ID NOs: 251-260 or 271. In another embodiment, the nucleobases of the antisense oligonucleotide as described herein are complementary to a portion of any one or more of 233-250 or 274 or SEQ ID NOs: 2298-2305 or SEQ ID NOs: 261-270 or 273.

In some embodiments, the antisense oligonucleotide as described herein comprises SEQ ID NOs: 1-25 or 53-82. In other embodiments, the antisense oligonucleotide as described herein comprises SEQ ID NOs: 3, 4, 7, 8, 13 or 19. In other embodiments, the antisense oligonucleotide as described herein comprises SEQ ID NOs: 54, 55, 56, 58, 59, or 80.

In some embodiments, the antisense oligonucleotide as described herein comprises SEQ ID NOs: 29-52 or 83-107. In other embodiments, the antisense oligonucleotide as described herein comprises SEQ ID NOs: 32 or 30 or 35. In other embodiments, the antisense oligonucleotide as described herein comprises SEQ ID NOs: 88, 91, 94, 95, 101, 104, 105, or 107.

In some embodiments, the antisense oligonucleotide as described herein comprises 14-22 nucleotides. In other embodiments, the antisense oligonucleotide as described herein comprises 14-25 nucleotides. In other embodiments, the antisense oligonucleotide as described herein comprises 15-25 nucleotides. In other embodiments, the antisense oligonucleotide as described herein comprises 16-24 nucleotides. In other embodiments, the antisense oligonucleotide as described herein comprises 17-22 nucleotides. In other embodiments, the antisense oligonucleotide as described herein comprises 17-21 nucleotides. In other embodiments, the antisense oligonucleotide as described herein comprises 18-21 nucleotides. In other embodiments, the antisense oligonucleotide as described herein comprises 15 nucleotides. In other embodiments, the antisense oligonucleotide as described herein comprises 16 nucleotides. In other embodiments, the antisense oligonucleotide as described herein comprises 17 nucleotides. In other embodiments, the antisense oligonucleotide as described herein comprises 18 nucleotides. In other embodiments, the antisense oligonucleotide as described herein comprises 19 nucleotides. In other embodiments, the antisense oligonucleotide as described herein comprises 20 nucleotides. In other embodiments, the antisense oligonucleotide as described herein comprises 21 nucleotides. In other embodiments, the antisense oligonucleotide as described herein comprises 22 nucleotides. In other embodiments, the antisense oligonucleotide as described herein comprises 23 nucleotides. In other embodiments, the antisense oligonucleotide as described herein comprises 24 nucleotides. In other embodiments, the antisense oligonucleotide as described herein comprises 25 nucleotides.

In other embodiments, the antisense oligonucleotide as described herein comprises any one of SEQ ID NOs: 1-107. In other embodiments, the antisense oligonucleotide as described herein has 14-22 nucleotides and comprises any one of SEQ ID NOs: 1-107.

In other embodiments, the antisense oligonucleotide as described herein comprises any one of SEQ ID NOs: 275-2290. In other embodiments, the antisense oligonucleotide as described herein has 14-22 nucleotides and comprises any one of SEQ ID NOs: 275-2290.

In other embodiments, the antisense oligonucleotide as described herein has 14-22 nucleotides and targets exons 4-8, 10-11, 13, 16-22, 26, 28-29, or 31 of MUC5AC, MUC5B, or both MUC5AC and MUC5B and/or surrounding intronic sequences.

In some embodiments, targeting of an exon as described herein comprises targeting (a) one or more sequences in the exon; (b) one or more portions of an intron adjacent to the exon; or (c) the intron-exon junction of the exon.

In some embodiments, an antisense oligonucleotide as described herein targets an exon of MUC5AC, MUC5B, or both MUC5AC and MUC5B. In other embodiments, an antisense oligonucleotide as described herein targets an intron of MUC5AC, MUC5B, or both MUC5AC and MUC5B. In other embodiments, an antisense oligonucleotide as described herein targets both an intron and an exon of MUC5AC, MUC5B, or both MUC5AC and MUC5B, which in one embodiment, is the intron-exon junction of an exon of MUC5AC, MUC5B, or both MUC5AC and MUC5B. In some embodiments, an antisense oligonucleotide as described herein targets a MUC5AC, MUC5B, or both MUC5AC and MUC5B exon and/or an intronic sequence that is adjacent to the exon.

In some embodiments, the intron that is adjacent to the exon is 5-60 nucleotides upstream or downstream of the exon. In other embodiments, the intron that is adjacent to the exon is 25-35 nucleotides upstream or downstream of the exon. In other embodiments, the intron that is adjacent to the exon is 1-100 nucleotides upstream or downstream of the exon. In other embodiments, the intron that is adjacent to the exon is 25-75 nucleotides upstream or downstream of the exon. In other embodiments, the intron that is adjacent to the exon is 10-50 nucleotides upstream or downstream of the exon. In other embodiments, the intron that is adjacent to the exon is 10-30 nucleotides upstream or downstream of the exon. In other embodiments, the intron that is adjacent to the exon is 5-20 nucleotides upstream or downstream of the exon. In other embodiments, the intron that is adjacent to the exon is 1-50 nucleotides upstream or downstream of the exon. In other embodiments, the intron that is adjacent to the exon is 20-40 nucleotides upstream or downstream of the exon. In other embodiments, the intron that is adjacent to the exon is 15-45 nucleotides upstream or downstream of the exon.

In another embodiment, the antisense oligonucleotide as described herein is complementary to a portion of Exon 4 of MUC5AC. In one embodiment, the antisense oligonucleotide is complementary to a portion of SEQ ID NO: 2290. In one embodiment, the antisense oligonucleotide is complementary to Exon 4 or the surrounding nucleotides. In one embodiment, the antisense oligonucleotide is complementary to a portion of SEQ ID NO: 215.

In another embodiment, the antisense oligonucleotide as described herein is complementary to a portion of Exon 5 of MUC5AC. In one embodiment, the antisense oligonucleotide is complementary to a portion of SEQ ID NO: 2291. In one embodiment, the antisense oligonucleotide is complementary to Exon 5 or the surrounding nucleotides. In one embodiment, the antisense oligonucleotide is complementary to a portion of SEQ ID NO: 216.

In another embodiment, the antisense oligonucleotide as described herein is complementary to a portion of Exon 6 of MUC5AC. In one embodiment, the antisense oligonucleotide is complementary to a portion of SEQ ID NO: 2292. In one embodiment, the antisense oligonucleotide is complementary to Exon 6 or the surrounding nucleotides. In one embodiment, the antisense oligonucleotide is complementary to a portion of SEQ ID NO: 217.

In another embodiment, the antisense oligonucleotide as described herein is complementary to a portion of Exon 7 of MUC5AC. In one embodiment, the antisense oligonucleotide is complementary to a portion of SEQ ID NO: 2293. In one embodiment, the antisense oligonucleotide is complementary to Exon 7 or the surrounding nucleotides. In one embodiment, the antisense oligonucleotide is complementary to a portion of SEQ ID NO: 218.

In another embodiment, the antisense oligonucleotide as described herein is complementary to a portion of Exon 8 of MUC5AC. In one embodiment, the antisense oligonucleotide is complementary to a portion of SEQ ID NO: 2294. In one embodiment, the antisense oligonucleotide is complementary to Exon 8 or the surrounding nucleotides. In one embodiment, the antisense oligonucleotide is complementary to a portion of SEQ ID NO: 219.

In another embodiment, the antisense oligonucleotide as described herein is complementary to a portion of Exon 10 of MUC5AC. In one embodiment, the antisense oligonucleotide is complementary to a portion of SEQ ID NO: 2295. In one embodiment, the antisense oligonucleotide is complementary to Exon 10 or the surrounding nucleotides. In one embodiment, the antisense oligonucleotide is complementary to a portion of SEQ ID NO: 220.

In another embodiment, the antisense oligonucleotide as described herein is complementary to a portion of Exon 11 of MUC5AC. In one embodiment, the antisense oligonucleotide is complementary to a portion of SEQ ID NO: 2296. In one embodiment, the antisense oligonucleotide is complementary to Exon 11 or the surrounding nucleotides. In one embodiment, the antisense oligonucleotide is complementary to a portion of SEQ ID NO: 221.

In another embodiment, the antisense oligonucleotide as described herein is complementary to a portion of Exon 13 of MUC5AC. In one embodiment, the antisense oligonucleotide is complementary to a portion of SEQ ID NO: 2297. In one embodiment, the antisense oligonucleotide is complementary to Exon 13 or the surrounding nucleotides. In one embodiment, the antisense oligonucleotide is complementary to a portion of SEQ ID NO: 222.

In another embodiment, the antisense oligonucleotide as described herein is complementary to a portion of Exon 16 of MUC5AC. In one embodiment, the antisense oligonucleotide is complementary to a portion of SEQ ID NO: 251. In one embodiment, the antisense oligonucleotide is complementary to Exon 16 or the surrounding nucleotides. In one embodiment, the antisense oligonucleotide is complementary to a portion of SEQ ID NO: 223.

In another embodiment, the antisense oligonucleotide as described herein is complementary to a portion of Exon 17 of MUC5AC. In one embodiment, the antisense oligonucleotide is complementary to a portion of SEQ ID NO: 252. In one embodiment, the antisense oligonucleotide is complementary to Exon 17 or the surrounding nucleotides. In one embodiment, the antisense oligonucleotide is complementary to a portion of SEQ ID NO: 224.

In another embodiment, the antisense oligonucleotide as described herein is complementary to a portion of Exon 18 of MUC5AC. In one embodiment, the antisense oligonucleotide is complementary to a portion of SEQ ID NO: 253. In one embodiment, the antisense oligonucleotide is complementary to Exon 18 or the surrounding nucleotides. In one embodiment, the antisense oligonucleotide is complementary to a portion of SEQ ID NO: 225.

In another embodiment, the antisense oligonucleotide as described herein is complementary to a portion of Exon 19 of MUC5AC. In one embodiment, the antisense oligonucleotide is complementary to a portion of SEQ ID NO: 254. In one embodiment, the antisense oligonucleotide is complementary to Exon 19 or the surrounding nucleotides. In one embodiment, the antisense oligonucleotide is complementary to a portion of SEQ ID NO: 226.

In another embodiment, the antisense oligonucleotide as described herein is complementary to a portion of Exon 20 of MUC5AC. In one embodiment, the antisense oligonucleotide is complementary to a portion of SEQ ID NO: 255. In one embodiment, the antisense oligonucleotide is complementary to Exon 20 or the surrounding nucleotides. In one embodiment, the antisense oligonucleotide is complementary to a portion of SEQ ID NO: 227.

In another embodiment, the antisense oligonucleotide as described herein is complementary to a portion of Exon 21 of MUC5AC. In one embodiment, the antisense oligonucleotide is complementary to a portion of SEQ ID NO: 256. In one embodiment, the antisense oligonucleotide is complementary to Exon 21 or the surrounding nucleotides. In one embodiment, the antisense oligonucleotide is complementary to a portion of SEQ ID NO: 228.

In another embodiment, the antisense oligonucleotide as described herein is complementary to a portion of Exon 22 of MUC5AC. In one embodiment, the antisense oligonucleotide is complementary to a portion of SEQ ID NO: 257. In one embodiment, the antisense oligonucleotide is complementary to Exon 22 or the surrounding nucleotides. In one embodiment, the antisense oligonucleotide is complementary to a portion of SEQ ID NO: 229.

In another embodiment, the antisense oligonucleotide as described herein is complementary to a portion of Exon 26 of MUC5AC. In one embodiment, the antisense oligonucleotide is complementary to a portion of SEQ ID NO: 258. In one embodiment, the antisense oligonucleotide is complementary to Exon 26 or the surrounding nucleotides. In one embodiment, the antisense oligonucleotide is complementary to a portion of SEQ ID NO: 230.

In another embodiment, the antisense oligonucleotide as described herein is complementary to a portion of Exon 28 of MUC5AC. In one embodiment, the antisense oligonucleotide is complementary to a portion of SEQ ID NO: 259. In one embodiment, the antisense oligonucleotide is complementary to Exon 28 or the surrounding nucleotides. In one embodiment, the antisense oligonucleotide is complementary to a portion of SEQ ID NO: 231.

In another embodiment, the antisense oligonucleotide as described herein is complementary to a portion of Exon 29 of MUC5AC. In one embodiment, the antisense oligonucleotide is complementary to a portion of SEQ ID NO: 260. In one embodiment, the antisense oligonucleotide is complementary to Exon 29 or the surrounding nucleotides. In one embodiment, the antisense oligonucleotide is complementary to a portion of SEQ ID NO: 232.

In another embodiment, the antisense oligonucleotide as described herein is complementary to a portion of Exon 31 of MUC5AC. In one embodiment, the antisense oligonucleotide is complementary to a portion of SEQ ID NO: 271. In one embodiment, the antisense oligonucleotide is complementary to Exon 31 or the surrounding nucleotides. In one embodiment, the antisense oligonucleotide is complementary to a portion of SEQ ID NO: 272.

In another embodiment, the antisense oligonucleotide as described herein is complementary to a portion of Exon 4 of MUC5B. In one embodiment, the antisense oligonucleotide is complementary to a portion of SEQ ID NO: 2298. In one embodiment, the antisense oligonucleotide is complementary to Exon 4 or the surrounding nucleotides. In one embodiment, the antisense oligonucleotide is complementary to a portion of SEQ ID NO: 233.

In another embodiment, the antisense oligonucleotide as described herein is complementary to a portion of Exon 5 of MUC5B. In one embodiment, the antisense oligonucleotide is complementary to a portion of SEQ ID NO: 2299. In one embodiment, the antisense oligonucleotide is complementary to Exon 5 or the surrounding nucleotides. In one embodiment, the antisense oligonucleotide is complementary to a portion of SEQ ID NO: 234.

In another embodiment, the antisense oligonucleotide as described herein is complementary to a portion of Exon 6 of MUC5B. In one embodiment, the antisense oligonucleotide is complementary to a portion of SEQ ID NO: 2300. In one embodiment, the antisense oligonucleotide is complementary to Exon 6 or the surrounding nucleotides. In one embodiment, the antisense oligonucleotide is complementary to a portion of SEQ ID NO: 235.

In another embodiment, the antisense oligonucleotide as described herein is complementary to a portion of Exon 7 of MUC5B. In one embodiment, the antisense oligonucleotide is complementary to a portion of SEQ ID NO: 2301. In one embodiment, the antisense oligonucleotide is complementary to Exon 7 or the surrounding nucleotides. In one embodiment, the antisense oligonucleotide is complementary to a portion of SEQ ID NO: 236.

In another embodiment, the antisense oligonucleotide as described herein is complementary to a portion of Exon 8 of MUC5B. In one embodiment, the antisense oligonucleotide is complementary to a portion of SEQ ID NO: 2302. In one embodiment, the antisense oligonucleotide is complementary to Exon 8 or the surrounding nucleotides. In one embodiment, the antisense oligonucleotide is complementary to a portion of SEQ ID NO: 237.

In another embodiment, the antisense oligonucleotide as described herein is complementary to a portion of Exon 10 of MUC5B. In one embodiment, the antisense oligonucleotide is complementary to a portion of SEQ ID NO: 2303. In one embodiment, the antisense oligonucleotide is complementary to Exon 10 or the surrounding nucleotides. In one embodiment, the antisense oligonucleotide is complementary to a portion of SEQ ID NO: 238.

In another embodiment, the antisense oligonucleotide as described herein is complementary to a portion of Exon 11 of MUC5B. In one embodiment, the antisense oligonucleotide is complementary to a portion of SEQ ID NO: 2304. In one embodiment, the antisense oligonucleotide is complementary to Exon 11 or the surrounding nucleotides. In one embodiment, the antisense oligonucleotide is complementary to a portion of SEQ ID NO: 239.

In another embodiment, the antisense oligonucleotide as described herein is complementary to a portion of Exon 13 of MUC5B. In one embodiment, the antisense oligonucleotide is complementary to a portion of SEQ ID NO: 2305. In one embodiment, the antisense oligonucleotide is complementary to Exon 13 or the surrounding nucleotides. In one embodiment, the antisense oligonucleotide is complementary to a portion of SEQ ID NO: 240.

In another embodiment, the antisense oligonucleotide as described herein is complementary to a portion of Exon 16 of MUC5B. In one embodiment, the antisense oligonucleotide is complementary to a portion of SEQ ID NO: 261. In one embodiment, the antisense oligonucleotide is complementary to Exon 16 or the surrounding nucleotides. In one embodiment, the antisense oligonucleotide is complementary to a portion of SEQ ID NO: 241.

In another embodiment, the antisense oligonucleotide as described herein is complementary to a portion of Exon 17 of MUC5B. In one embodiment, the antisense oligonucleotide is complementary to a portion of SEQ ID NO: 262. In one embodiment, the antisense oligonucleotide is complementary to Exon 17 or the surrounding nucleotides. In one embodiment, the antisense oligonucleotide is complementary to a portion of SEQ ID NO: 242.

In another embodiment, the antisense oligonucleotide as described herein is complementary to a portion of Exon 18 of MUC5B. In one embodiment, the antisense oligonucleotide is complementary to a portion of SEQ ID NO: 263. In one embodiment, the antisense oligonucleotide is complementary to Exon 18 or the surrounding nucleotides. In one embodiment, the antisense oligonucleotide is complementary to a portion of SEQ ID NO: 243.

In another embodiment, the antisense oligonucleotide as described herein is complementary to a portion of Exon 19 of MUC5B. In one embodiment, the antisense oligonucleotide is complementary to a portion of SEQ ID NO: 264. In one embodiment, the antisense oligonucleotide is complementary to Exon 19 or the surrounding nucleotides. In one embodiment, the antisense oligonucleotide is complementary to a portion of SEQ ID NO: 244.

In another embodiment, the antisense oligonucleotide as described herein is complementary to a portion of Exon 20 of MUC5B. In one embodiment, the antisense oligonucleotide is complementary to a portion of SEQ ID NO: 265. In one embodiment, the antisense oligonucleotide is complementary to Exon 20 or the surrounding nucleotides. In one embodiment, the antisense oligonucleotide is complementary to a portion of SEQ ID NO: 245.

In another embodiment, the antisense oligonucleotide as described herein is complementary to a portion of Exon 21 of MUC5B. In one embodiment, the antisense oligonucleotide is complementary to a portion of SEQ ID NO: 266. In one embodiment, the antisense oligonucleotide is complementary to Exon 21 or the surrounding nucleotides. In one embodiment, the antisense oligonucleotide is complementary to a portion of SEQ ID NO: 246.

In another embodiment, the antisense oligonucleotide as described herein is complementary to a portion of Exon 22 of MUC5B. In one embodiment, the antisense oligonucleotide is complementary to a portion of SEQ ID NO: 267. In one embodiment, the antisense oligonucleotide is complementary to Exon 22 or the surrounding nucleotides. In one embodiment, the antisense oligonucleotide is complementary to a portion of SEQ ID NO: 247.

In another embodiment, the antisense oligonucleotide as described herein is complementary to a portion of Exon 26 of MUC5B. In one embodiment, the antisense oligonucleotide is complementary to a portion of SEQ ID NO: 268. In one embodiment, the antisense oligonucleotide is complementary to Exon 26 or the surrounding nucleotides. In one embodiment, the antisense oligonucleotide is complementary to a portion of SEQ ID NO: 248.

In another embodiment, the antisense oligonucleotide as described herein is complementary to a portion of Exon 28 of MUC5B. In one embodiment, the antisense oligonucleotide is complementary to a portion of SEQ ID NO: 269. In one embodiment, the antisense oligonucleotide is complementary to Exon 28 or the surrounding nucleotides. In one embodiment, the antisense oligonucleotide is complementary to a portion of SEQ ID NO: 249.

In another embodiment, the antisense oligonucleotide as described herein is complementary to a portion of Exon 29 of MUC5B. In one embodiment, the antisense oligonucleotide is complementary to a portion of SEQ ID NO: 270. In one embodiment, the antisense oligonucleotide is complementary to Exon 29 or the surrounding nucleotides. In one embodiment, the antisense oligonucleotide is complementary to a portion of SEQ ID NO: 250.

In another embodiment, the antisense oligonucleotide as described herein is complementary to a portion of Exon 31 of MUC5B. In one embodiment, the antisense oligonucleotide is complementary to a portion of SEQ ID NO: 273. In one embodiment, the antisense oligonucleotide is complementary to Exon 31 or the surrounding nucleotides. In one embodiment, the antisense oligonucleotide is complementary to a portion of SEQ ID NO: 274.

In some embodiments, the ASOs as described herein are targeted to portions of MUC5AC or MUC5B which are either unique to MUC5AC or MUC5B or common to MUC5AC and MUC5B, leading to a decrease in mRNA and protein production. In some embodiments, the target is unique to MUC5AC. In other embodiments, the target is unique to MUC5B. In yet other embodiments, the target is common to MUC5AC and MUC5B sequences.

In some embodiments, the antisense oligonucleotide as described herein is complementary to a portion of exon 4, exon 17, exon 26, or exon 31 of both MUC5AC and MUC5B.

In some embodiments, the antisense oligonucleotide as described herein is complementary to a portion of both Exon 4 (and/or the surrounding nucleotides) of MUC5A and Exon 4 (and/or the surrounding nucleotides) of MUC5B. In one embodiment, the antisense oligonucleotide is complementary to a portion of any combination of SEQ ID NO: 2290, SEQ ID NO: 215, SEQ ID NO: 2298, and SEQ ID NO: 233.

In some embodiments, the antisense oligonucleotide as described herein is complementary to a portion of both Exon 17 (and/or the surrounding nucleotides) of MUC5A and Exon 17 (and/or the surrounding nucleotides) of MUC5B. In one embodiment, the antisense oligonucleotide is complementary to a portion of any combination of SEQ ID NO: 252, SEQ ID NO: 224, SEQ ID NO: 262, and SEQ ID NO: 242.

In some embodiments, the antisense oligonucleotide as described herein is complementary to a portion of both Exon 26 (and/or the surrounding nucleotides) of MUC5A and Exon 26 (and/or the surrounding nucleotides) of MUC5B. In one embodiment, the antisense oligonucleotide is complementary to a portion of any combination of SEQ ID NO: 258, SEQ ID NO: 230, SEQ ID NO: 268, and SEQ ID NO: 248.

In some embodiments, the antisense oligonucleotide as described herein is complementary to a portion of both Exon 31 (and/or the surrounding nucleotides) of MUC5A and Exon 26 (and/or the surrounding nucleotides) of MUC5B. In one embodiment, the antisense oligonucleotide is complementary to a portion of any combination of SEQ ID NO: 271, SEQ ID NO: 272, SEQ ID NO: 273, and SEQ ID NO: 274.

In other embodiments, the ASOs as described herein create a premature stop codon in the MUC5AC, MUC5B, or both MUC5A and MUC5B mRNA.

In some embodiments, the antisense oligonucleotide comprises at least 14 bases, at least 15 bases, at least 16 bases, at least 17 bases, at least 18 bases, at least 19 bases, at least 20 bases, at least 21 bases, at least 22 bases, at least 23 bases, at least 24 bases, or at least 25 bases, or any value and range therebetween. Each possibility represents a separate embodiment of the invention.

In some embodiments, the antisense oligonucleotide comprises 14 to 25 bases, 14 to 24 bases, 14 to 23 bases, 14 to 22 bases, 14 to 21 bases, 14 to 20 bases, 14 to 19 bases, or 14 to 18 bases, or 14 to 17 bases. Each possibility represents a separate embodiment of the invention. In some embodiments, the antisense oligonucleotide comprises 17 to 22 bases.

In some embodiments, the term "complementary" refers to the ability of polynucleotides to form base pairs with one another. Base pairs are typically formed by hydrogen bonds between nucleotide units in antiparallel polynucleotide strands. Complementary polynucleotide strands can base pair in the Watson-Crick manner (e.g., A to T, A to U, C to G), or in any other manner that allows for the formation of duplexes. As persons skilled in the art are aware, when using RNA as opposed to DNA, uracil rather than thymine is the base that is considered to be complementary to adenosine. However, when a U is denoted in the context of the present invention, the ability to substitute a T is implied, unless otherwise stated.

In some embodiments, antisense oligonucleotides target nucleic acids and may comprise chemical modifications and motifs. Antisense oligonucleotides may modulate protein expression by binding to a target messenger pre-mRNA encoding the protein. In some embodiments, the binding of an antisense oligonucleotide to its target mRNA modulates the processing of pre-mRNA. In some embodiments, antisense oligonucleotides alter splicing of a pre-mRNA. In some embodiments, antisense oligonucleotides enhance skipping of one or more MUC5AC, MUC5B, or both MUC5AC and MUC5B exons. In other embodiments, antisense oligonucleotides reduce inclusion of one or more MUC5AC, MUC5B, or both MUC5AC and MUC5B exons.

As used herein, antisense oligonucleotide describes an oligonucleotide at least a portion of which is complementary to a target nucleic acid to which it is capable of hybridizing, resulting in at least one antisense activity.

As used herein, "antisense activity" describes any detectable and/or measurable change attributable to the hybridization of an antisense compound to its target nucleic acid. As used herein, "detecting" or "measuring" means that a test or assay for detecting or measuring is performed as reflected by changes in RNA length, sequence and amount. Such detection and/or measuring may result in a value of zero. Thus, if a test for detection or measuring results in a finding of no activity (activity of zero), the step of detecting or measuring the activity has nevertheless been performed.

In some embodiments, the antisense oligonucleotide comprises a chemical modification. In some embodiments, the chemical modification comprises a modification of a backbone of the antisense oligonucleotide. In some embodiments, the antisense oligonucleotide comprises one or more phosphate-ribose. In other embodiments, the antisense oligonucleotide comprises one or more phosphate-deoxyriboses. In other embodiments, the antisense oligonucleotide comprises one or more phosphorothioates. In other embodiments, the antisense oligonucleotide comprises one or more phosphorothioate-deoxyriboses. In other embodiments, the antisense oligonucleotide comprises one or more 2'-O-methyl-phosphorothioates (2OMPs). In other embodiments, the antisense oligonucleotide comprises one or more phosphorodiamidates morpholino (PMOs). In other embodiments, the antisense oligonucleotide comprises one or more peptide nucleic acids (PNAs). In other embodiments, the antisense oligonucleotide comprises one or more 2-methoxyethyl phosphorothioates (MOEs). In other embodiments, the antisense oligonucleotide comprises a constrained ethyl (cET) backbone. In other embodiments, the antisense oligonucleotide comprises an alternating locked nucleic acid (LNA) backbone. In other embodiments, the antisense oligonucleotide comprises one or more N3'-P5' phosphoramidates. In other embodiments, the antisense oligonucleotide comprises one or more 2'-deoxy-2'-fluoro-β-d-arabino nucleic acids. In other embodiments, the antisense oligonucleotide comprises one or more cyclohexene nucleic acids (CeNAs). In other embodiments, the antisense oligonucleotide comprises one or more tricyclo-DNA (tcDNA) nucleic acids. In other embodiments, the antisense oligonucleotide comprises one or more 2-amino-2'-deoxyadenosines (DAPs). In other embodiments, the antisense oligonucleotide comprises one or more 2'-deoxy-2'-fluoro-β-D-arabinonucleic acids (FANAs). In other embodiments, the antisense oligonucleotide comprises a ligand-conjugated antisense (LICA). In other embodiments, the antisense oligonucleotide comprises a combination of any of the chemical modifications described herein.

In some embodiments, the chemical modification is a modification of a sugar of the antisense oligonucleotide. In some embodiments, the sugar modification comprises 2'-modified and conformationally constrained nucleotides. In other embodiments, the chemical modification comprises a 2'-fluoro modification. Thus, in some embodiments, the modified oligonucleotide comprises at least one modified sugar. In some embodiments, the at least one modified sugar is a bicyclic sugar, such as LNA, ENA or cEt. In some embodiments, the at least one modified sugar comprises a 2'-modified sugar moiety, such as 2'-O-methyl, 2'-F, 2'-O-methylethyl, or 2'-O-methoxyethyl.

In some embodiments, the chemical modification is a modification of a nucleobase of the antisense oligonucleotide. In some embodiments, the chemical modification is a modification of the 3' terminal. In some embodiments, the chemical modification is a modification of the 5' terminal. In some embodiment, the chemical modification is a combination of two or more of the modifications as described herein. In some embodiments, the oligonucleotide used in the compositions and methods as described herein is chimeric. In some embodiments, the oligonucleotide used in the compositions and methods as described herein comprises multiple types of modified nucleotides. In some embodiments, the oligonucleotide comprises mixtures of LNA/DNA or LNA/2'OMe/MOE-RNA. In other embodiments, the oligonucleotide comprises combinations of 2-thiothymidine, 3'-fluorohexitol nucleic acid (FHNA), cEt, a 5-modified pyrimidine base, and α,β constrained nucleic acid (α,β-CNA). In some embodiments, the oligonucleotide comprises Fluoroarabinonucleic acid (2'F-ANA).

In some embodiments, the oligonucleotide is a modified oligonucleotide. In some embodiments, the modified oligonucleotide comprises a nucleoside analogue modification, a modified nucleobase, a modified internucleoside linkage a modified sugar, or a combination thereof. In some embodiments, the modified oligonucleotide comprises at least one nucleoside analogue. In some embodiments, the modified oligonucleotide comprises at least one modified nucleobase. In some embodiments the at least one modified nucleobase is 5-methyl-cytosine. In some embodiments, the modified oligonucleotide comprises at least one modified internucleoside linkage. In some embodiments, the at least one modified internucleoside linkage is a phosphothioester internucleoside linkage.

In some embodiments, the modified oligonucleotide is a nucleic acid analogue, such as a peptide nucleic acid (PNA) or a morpholino.

In some embodiments, oligonucleotides are modified by attachment of one or more conjugate groups. In general, conjugate groups modify one or more properties of the attached oligonucleotide including but not limited to pharmacodynamics, pharmacokinetics, stability, binding, absorption, cellular distribution, cellular uptake, charge and clearance. Conjugate groups are linked directly or via an optional conjugate linking moiety or conjugate linking group to a parent compound such as an oligonucleotide. A conjugate group as described herein comprises intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins, dyes or a combination thereof.

In some embodiments, a synthetic antisense oligonucleotide as described herein is fully chemically modified. In some embodiments, "fully chemical modified" as described herein describes an oligonucleotide in which the entire nucleotide sequence is chemically modified as described herein. In other embodiments, "fully chemical modified" indicates that all bases of an oligonucleotide are modified.

In other embodiments, a synthetic antisense oligonucleotide as described herein is comprised only of ribonucleic acids. In other embodiments, a synthetic antisense oligonucleotide as described herein lacks deoxyribonucleic acids. In other embodiments, a synthetic antisense oligonucleotide as described herein lacks DNA bases.

In some embodiments, oligonucleotides of the present invention that are targeted to a MUC5A, MUC5B, or both MUC5A and MUC5B nucleic acids have chemically modified subunits arranged in patterns, or motifs, to confer to the oligonucleotide properties such as enhanced inhibitory activity, increased binding affinity for a target nucleic acid, enhanced cellular uptake, or resistance to degradation by *in vivo* nucleases. In some embodiments, the chemically modified oligonucleotides may have a modification in one or more regions.

In some embodiments, the oligonucleotide comprises a gapmer, which, in one embodiment, is a DNA antisense oligonucleotide structures with RNA-like segments on both sides of the sequence. In some embodiments, the gapmer antisense oligonucleotide is 12-22 nucleotides. In other embodiments, the oligonucleotide as described herein is not a gapmer.

In some embodiments, oligonucleotides may also be modified to have one or more stabilizing groups that are generally attached to one or both termini of the oligonucleotide to enhance properties such as, for example, nuclease stability. In some embodiments, the stabilizing group comprises a cap structure. In some embodiments, the terminal modifications protect the oligonucleotide from exonuclease degradation, and facilitate delivery and/or localization within a cell. In some embodiments, the cap is present at the 5'-terminus (5'-cap), at the 3'-terminus (3'-cap), or on both termini. Cap structures are well known in the art and include, for example, inverted deoxy basic caps.

In some embodiments, the chemical modification increases stability of the antisense oligonucleotide in a cell. In some embodiments, the chemical modification increases stability of the antisense oligonucleotide *in vivo.* In some embodiments, the chemical modification increases the antisense oligonucleotide's ability to modulate splicing. In some embodiments, the chemical modification increases the functional output of the antisense nucleotide. In some embodiments, the chemical modification increases the half-life of the antisense oligonucleotide. In some embodiments, the chemical modification inhibits polymerase extension from the 3' end of the antisense oligonucleotide. In some embodiments, the chemical modification inhibits recognition of the antisense oligonucleotide by a polymerase.

In some embodiments, an oligonucleotide may have a sequence that has greater than 30% G-C content, greater than 40% G-C content, greater than 50% G-C content, greater than 60% G-C content, greater than 70% G-C content, or greater than 80% G-C content. An oligonucleotide may have a sequence that has up to 100% G-C content, up to 95% G-C content, up to 90% G-C content, or up to 80% G-C content. In some embodiments, the G-C content of an oligonucleotide is preferably between about 30-60%.

In some embodiments, a naked antisense oligonucleotide is delivered to cells. In other embodiments, the antisense oligonucleotide is bioconjugated or delivered via a nanocarrier. In some embodiment, the antisense oligonucleotide is non-covalently complexed with cationic polymers (for example, polyethylenimine), dendrimers, CPPs (for example, MPG-8, PepFect6, RVG-9R228, and Xentry-KALA). In other embodiments, the nanocarrier is a nanoparticle, which, in some embodiments, comprises a lipid nanoparticle. In some embodiments, a suitable lipid nanoparticle for the present invention comprises one or more of a cationic lipid, a non-cationic lipid, a cholesterol-based lipid, a PEG-modified lipid, an amphiphilic block copolymer and/or a polymer, or a combination thereof. In some embodiments, a lipid nanoparticle as described herein comprises a cationic lipid, DOPE or DEPE (non-cationic lipids), and DMG-PEG2K (PEG-modified lipid).

In some embodiments, the delivery potential of antisense oligonucleotides is enhanced through direct covalent conjugation of various moieties that promote intracellular uptake, target the drug to specific cells/tissues or reduce clearance from the circulation. These include lipids (for example, cholesterol that facilitates interactions with lipoprotein particles in the circulation), peptides (for cell targeting and/or cell penetration), aptamers, antibodies, and sugars (for example, N-acetylgalactosamine (GalNAc)).

In some embodiments, the antisense oligonucleotide is covalently conjugated to cell-targeting or cell-penetrating moieties. In some embodiments, the antisense oligonucleotide is delivered as part of an endogenous vesicle (exosome) loading. In other embodiments, the antisense oligonucleotide is delivered on spherical nucleic acids (SNAs). In other embodiments, nanotechnology applications (for example, DNA cages) and 'smart' materials are used for antisense oligonucleotide delivery.

In some embodiments, the antisense oligonucleotide is devoid of a labeling moiety. In some embodiments, the antisense oligonucleotide is not labeled. In some embodiments, the antisense oligonucleotide does not emit a detectable signal or does not comprise moieties capable of being recognized so as to enable nucleic acid detection (e.g., digoxigenin and fluorescently labeled anti-DIG antibody). In some embodiments, a detectable signal comprises a dye or an emitting energy which provides detection of a compound, e.g., a polynucleotide, *in vivo* or *in vitro.* In some embodiments, a detectable signal comprises: a fluorescent signal, a chromatic signal, or a radioactive signal.

In some embodiments, the antisense oligonucleotide is devoid of radioactive nucleobase(s); digoxigenin, streptavidin, biotin, a fluorophore, hapten label, CLICK label, amine label, or thiol label.

In other embodiments, the antisense oligonucleotide comprises a labeling moiety or a radioactive nucleobase as described hereinabove.

In some embodiments, the pre-mRNA to which the antisense oligonucleotide is targeted is wildtype pre-mRNA. In other embodiments, the pre-mRNA to which the antisense oligonucleotide is targeted is mutated pre-mRNA.

In some embodiments, the antisense oligonucleotide is specific to a MUC5AC, MUC5B, or both MUC5AC and MUC5B pre-mRNA. In some embodiments, the term "specific" refers to base pair specificity, gene specificity, or to both base pair and gene specificity. In some embodiments, the antisense oligonucleotide is specific to the MUC5AC, MUC5B, or both MUC5AC and MUC5B gene. In some embodiments, the antisense oligonucleotide masks a splice enhancing motif or region of MUC5AC, MUC5B, or both MUC5AC and MUC5B. In other embodiments, the antisense oligonucleotide enhances a splice silencing motif or region of MUC5AC, MUC5B, or both MUC5AC and MUC5B.

In some embodiments, the antisense oligonucleotide binds the MUC5AC, MUC5B, or both MUC5AC and MUC5B pre-mRNA with perfect or 100% complementarity. In other embodiments, the antisense oligonucleotide binds the MUC5AC, MUC5B, or both MUC5AC and MUC5B pre-mRNA with greater than 90% complementarity. In other embodiments, the antisense oligonucleotide binds the MUC5AC, MUC5B, or both MUC5AC and MUC5B pre-mRNA with greater than 95% complementarity. In other embodiments, the antisense oligonucleotide binds the MUC5AC, MUC5B, or both MUC5AC and MUC5B pre-mRNA with greater than 98% complementarity. In other embodiments, the antisense oligonucleotide binds the MUC5AC, MUC5B, or both MUC5AC and MUC5B pre-mRNA with greater than 99% complementarity. Each possibility represents a separate embodiment of the invention.

In one embodiment, the BLAST algorithm may be used to identify regions of similarity amongst different nucleic acids. In some embodiments, a comparison of the sequence of a target nucleic acid to other sequences throughout the genome may prevent the selection of antisense strand sequences that may hybridize in a non-specific manner to sequences other than a selected target nucleic acid (i.e., non-target or off-target sequences).

In some embodiments, the antisense oligonucleotide does not bind any gene product other than MUC5AC, MUC5B, or both MUC5AC and MUC5B with perfect complementarity. In some embodiments, the antisense oligonucleotide does not bind any gene other than MUC5AC, MUC5B, or both MUC5AC and MUC5B with a complementarity of greater than 70, 75, 80, 85, 90, 95, 97, or99%. Each possibility represents a separate embodiment of the invention. In some embodiments, the antisense oligonucleotide does not bind any gene other than MUC5AC, MUC5B, or both MUC5AC and MUC5B with a complementarity of greater than 90%.

In some embodiments, the antisense oligonucleotide does not bind with perfect complementarity to anywhere in the genome of a cell other than within MUC5AC, MUC5B, or both MUC5AC and MUC5B. In some embodiments, the antisense oligonucleotide does not bind with complementarity of greater than 70, 75, 80, 85, 90, 95, 97, or 99% to anywhere in the genome of a cell other than within MUC5AC, MUC5B, or both MUC5AC and MUC5B. Each possibility represents a separate embodiment of the invention.

In some embodiments, a mutated pre-mRNA comprises at least one mutation in an exon of the MUC5AC, MUC5B, or both MUC5AC and MUC5B pre-mRNA. In other embodiments, a mutated pre-mRNA comprises at least one mutation in an intron of the MUC5AC, MUC5B, or both MUC5AC and MUC5B pre-mRNA.

In some embodiments, the antisense oligonucleotide is specific to a MUC5AC, MUC5B, or both MUC5AC and MUC5B pre-mRNA. In some embodiments, the term "specific" refers to base pair specificity, gene specificity, or to both base pair and gene specificity. In some embodiments, the antisense oligonucleotide is specific to the MUC5AC, MUC5B, or both MUC5AC and MUC5B gene. In some embodiments, the antisense oligonucleotide masks a splice enhancing motif, sequence, or region of MUC5AC, MUC5B, or both MUC5AC and MUC5B. In some embodiments, the antisense oligonucleotide enhances exon skipping. In some embodiments, the antisense oligonucleotide inhibits exon inclusion.

In some embodiments, the antisense oligonucleotide binds the MUC5AC, MUC5B, or both MUC5AC and MUC5B pre-mRNA with perfect or 100% complementarity. In other embodiments, the antisense oligonucleotide binds the MUC5AC, MUC5B, or both MUC5AC and MUC5B pre-mRNA with greater than 90% complementarity. In other embodiments, the antisense oligonucleotide binds the MUC5AC, MUC5B, or both MUC5AC and MUC5B pre-mRNA with greater than 95% complementarity. In other embodiments, the antisense oligonucleotide binds the MUC5AC, MUC5B, or both MUC5AC and MUC5B pre-mRNA with greater than 98% complementarity. In other embodiments, the antisense oligonucleotide binds the MUC5AC, MUC5B, or both MUC5AC and MUC5B pre-mRNA with greater than 99% complementarity. In other embodiments, the nucleobase sequence of the antisense strand of the oligonucleotide of the invention is at least 80%, at least 85%, at least 90%, at least 91%, at least 92%, at least 93%, at least 94%, at least 95%, at least 96%, at least 97%, at least 98%, at least 99%, or 100% complementary to an equal length portion of MUC5AC, MUC5B, or both MUC5AC and MUC5B pre-mRNA. Each possibility represents a separate embodiment of the invention.

In some embodiments, the antisense oligonucleotide does not bind any gene product other than MUC5AC, MUC5B, or both MUC5AC and MUC5B with perfect complementarity. In some embodiments, the antisense oligonucleotide does not bind any gene other than MUC5AC, MUC5B, or both MUC5AC and MUC5B with a complementarity of greater than 70, 75, 80, 85, 90, 95, 97, or 99%. Each possibility represents a separate embodiment of the invention. In some embodiments, the antisense oligonucleotide does not bind any gene other than MUC5AC, MUC5B, or both MUC5AC and MUC5B with a complementarity of greater than 90%.

In some embodiments, the antisense oligonucleotide does not bind with perfect complementarity to anywhere in the genome of a cell other than within MUC5AC, MUC5B, or both MUC5AC and MUC5B. In some embodiments, the antisense oligonucleotide does not bind with complementarity of greater than 70, 75, 80, 85, 90, 95, 97, or 99% to anywhere in the genome of a cell other than within MUC5AC, MUC5B, or both MUC5AC and MUC5B. Each possibility represents a separate embodiment of the invention. In some embodiments, the cell is a mammalian cell. In some embodiments, the mammal is a human.

In some embodiments, a mutated pre-mRNA comprises at least one mutation in an exon of the MUC5AC, MUC5B, or both MUC5AC and MUC5B pre-mRNA. In other embodiments, a mutated pre-mRNA comprises at least one mutation in an intron of the MUC5AC, MUC5B, or both MUC5AC and MUC5B pre-mRNA.

In some embodiments, the mutation comprises an early signal for termination of translation or a premature termination codon. In some embodiments, the mutation comprises a frameshift mutation. In some embodiments, the mutation comprises a missense mutation. In other embodiments, the mutation comprises a nonsense mutation. In some embodiments, the mutation comprises a substitution mutation in the MUC5AC, MUC5B, or both MUC5AC and MUC5B encoding gene, pre-mRNA encoded therefrom, or protein product thereof.

In some embodiments, the antisense oligonucleotide binds to an intronic sequence in the MUC5AC, MUC5B, or both MUC5AC and MUC5B pre-mRNA. In some embodiments, the antisense oligonucleotide binds to an intronic sequence that is distant from an exon, an intron-exon junction, or both. In some embodiments, the binding of an antisense oligonucleotide to an intronic sequence in the MUC5AC, MUC5B, or both MUC5AC and MUC5B pre-mRNA enhances exon skipping. In some embodiments, the binding of an antisense oligonucleotide to an exon sequence in the MUC5AC, MUC5B, or both MUC5AC and MUC5B pre-mRNAreduces exon inclusion.

In some embodiments, distant comprises at least 50 base pairs (bp), at least 100 bp, at least 200 bp, at least 350 bp, at least 500 bp, at least 750 bp, at least 1,000 bp, at least 2,000 bp, at least 3,500 bp, at least 5,000 bp, at least 7,500 bp, or at least 10,000 bp upstream to an exon, an intron-exon junction, or both, or downstream to an exon, an intro-exon junction, or both, or any value and range therebetween. Each possibility represents a separate embodiment of the invention.

In some embodiments, the antisense oligonucleotide modulates expression of MUC5AC, MUC5B, or both MUC5AC and MUC5B. In some embodiments, the antisense oligonucleotide modulates splicing of MUC5AC, MUC5B, or both MUC5AC and MUC5B. In some embodiments, the antisense oligonucleotide modulates splicing of an exon of MUC5AC, MUC5B, or both MUC5AC and MUC5B.

In some embodiments, the antisense oligonucleotide does not fully or partially bind (e.g., complement) to a sequence within a gene other than MUC5AC or MUC5B. In other embodiments, although the antisense oligonucleotide may partially or fully bind (e.g., complement) to an intronic sequence within a gene other than MUC5AC or MUC5B, it does not induce splicing or modify transcription in the non-MUC5AC or MUC5B gene.

In some embodiments, the antisense oligonucleotide does not cause an off-target effect. In some embodiments, off-target is a target other than MUC5AC. In other embodiments, off-target is a target other than MUC5B. In other embodiments, off-target is a target other than MUC5AC and MUC5B. In some embodiments, off-target is a target other than splicing of an exon of MUC5AC, MUC5B, or both MUC5AC and MUC5B. In some embodiments, the antisense oligonucleotide does not substantially or significantly modulate expression of a gene other than MUC5AC, MUC5B, or both MUC5AC and MUC5B. In some embodiments, the antisense oligonucleotide does not substantially or significantly modulate splicing of a gene other than MUC5AC, MUC5B, or both MUC5AC and MUC5B. In some embodiments, the antisense oligonucleotide does not substantially or significantly modulate splicing of an exon other than an exon of MUC5AC, MUC5B, or both MUC5AC and MUC5B. In some embodiments, substantial modulation of expression is a change in expression of at least 5%, 10%, 15%, 20%, 25%, 30%, 35%, 40%, 45% or 50%. Each possibility represents a separate embodiment of the invention. In some embodiments, substantial modulation of expression is a change in expression of at least 20%.

In some embodiments, an antisense oligonucleotide as described herein, or a pharmaceutical composition comprising an antisense oligonucleotide as described herein, is used in the modulation of splicing of a MUC5AC, MUC5B, or both MUC5AC and MUC5B pre-mRNA transcribed from a MUC5AC, MUC5B, or both MUC5AC and MUC5B genes.

In some embodiments, the phrase "modulation of splicing" refers to affecting a change in the level of any RNA or mRNA variant produced by the MUC5AC, MUC5B, or both MUC5AC and MUC5B native pre-mRNA. For example, in some embodiments, modulation comprises causing a decrease in the level of abnormal MUC5AC, MUC5B, or both MUC5AC and MUC5B mRNA.

In other embodiments, modulation comprises causing a decrease in the level of normal, full-length MUC5AC, MUC5B, or both MUC5AC and MUC5B mRNA. In some embodiments, the normal mRNA comprises a non-mutated exon. In other embodiments, the MUC5AC, MUC5B, or both MUC5AC and MUC5B pre-mRNA is devoid of a mutation. In some embodiments, the MUC5AC, MUC5B, or both MUC5AC and MUC5B pre-mRNA is a wildtype pre-mRNA. In one embodiment, normal comprises wild-type and abnormal comprises non-wild-type.

In other embodiments, modulation comprises causing a decrease in the level of both normal and abnormal MUC5AC, MUC5B, or both MUC5AC and MUC5B mRNA.

It is therefore evident that any change in ratio between certain MUC5AC, MUC5B, or both MUC5AC and MUC5B splicing variants is also considered to be the result of splicing modulation. Each possibility represents a separate embodiment of the invention.

In some embodiments, antisense oligonucleotides as described herein are single-stranded.

In some embodiments, the antisense oligonucleotide as described herein comprises an active fragment of an ASO as described herein.

In some embodiments, the term "active fragment" refers to a fragment that is 100% identical to a contiguous portion of the full nucleotide sequence of the antisense oligonucleotide, providing that at least: 30%, 40%, 50%, 60%, 70%, 80% or 90% of the activity of the original antisense oligonucleotide nucleotide sequence is retained, or any value and range therebetween. Each possibility represents a separate embodiment of the present invention.

The present invention also provides a vector comprising one or more oligonucleotides as described herein. Examples of appropriate vectors include adenoviral, lentiviral, adeno-associated viral (AAV), poliovirus, herpes simplex virus (HSV), or murine Maloney-based viral vectors. In one embodiment, the vector is an adeno-associated virus vector. In some embodiments, these cassettes and vectors may be contained in a cell, such as a mammalian cell. In some embodiments, a non-human mammal may comprise the cassette or vector.

In some embodiments, the present invention also provides a cell comprising one or more oligonucleotides as described herein. In some embodiments, the cell comprises a mammalian cell. In some embodiments, the mammal is a human. In some embodiments, a cell as described herein comprises nucleic acid molecules, expression cassettes or vectors as described herein. In some embodiments, a non-human mammal comprises the nucleic acid molecules, expression cassettes or vectors as described herein.

In some embodiments, the cell is derived from the subject as described herein. In some embodiments, the cell comprises a cell line or a culture thereof. In some embodiments, the cell is an epithelial cell. In some embodiments, an epithelial cell comprises a respiratory epithelial cell. In some embodiments, a respiratory epithelial cell is derived from the upper respiratory system. In some embodiments, a respiratory epithelial cell is a ciliated columnar epithelial cell. In some embodiments, a respiratory epithelial cell is a ciliated pseudo stratified columnar epithelial cell. In some embodiments, a respiratory epithelial cell is selected from: a ciliated cell, a goblet cell, a club cell, an airway basal cell, or any combination thereof. In other embodiments, an epithelial cell comprises a nasal epithelial cell. In other embodiments, an epithelial cell comprises a bronchial epithelial cell.

In some embodiments, the present invention provides a nucleic acid, an expression cassette, a vector, or a composition as described herein for use in therapy, such as for treating a lung disease or disorder, as described herein.

In some embodiments, oligonucleotides as described herein may hybridize to a MUC5AC or MUC5B nucleic acid in any stage of RNA processing. For example, described herein are oligonucleotides that are complementary to a pre-mRNA. Additionally, oligonucleotides described herein may hybridize to any element of a MUC5AC or MUC5B nucleic acid. For example, described herein are oligonucleotides that are complementary to an exon, an intron, the 5' UTR, the 3' UTR, a repeat region, a splice junction, an exon:exon splice junction, an exonic splicing silencer, or an exonic splicing enhancer.

In some embodiments, a MUC5 antisense oligonucleotide as described herein is targeted to a respiratory epithelial cell, which in one embodiment, comprises a ciliated cell, a goblet cell, a club cell, an airway basal cell, or any combination thereof.

### Pharmaceutical Compositions

### Formulations

According to some embodiments, the present invention provides a composition comprising an antisense oligonucleotide as described herein.

In some embodiments, the composition further comprises one or more non-toxic, pharmaceutically acceptable carriers and/or diluents and/or adjuvants (collectively referred to herein as "carrier" materials) and, if desired, other active ingredients.

The term "pharmaceutically acceptable carrier" in some embodiments refers to any of the standard pharmaceutical carriers known in the field such as sterile solutions, tablets, coated tablets, and capsules. Typically, such carriers contain excipients such as starch, milk, sugar, certain types of clay, gelatin, stearic acids, or salts thereof, magnesium or calcium stearate, talc, vegetable fats or oils, gums, glycols, or other known excipients. Such carriers may also include flavor and color additives or other ingredients. Examples of pharmaceutically acceptable carriers include, but are not limited to: water, saline, buffers, inert, nontoxic solids (e.g., mannitol, talc). In some embodiments, the diluent or carrier is as an aqueous solution. In some embodiments, the solution may include a buffer, which in some embodiments, comprises phosphate -buffered saline (PBS). Compositions comprising such carriers are formulated by well-known conventional methods. Depending on the intended mode of administration and the intended use, the compositions may be in the form of solid, semi-solid, or liquid dosage forms, such, for example, as powders, granules, crystals, liquids, suspensions, liposomes, nanoparticles, nano-emulsions, pastes, creams, salves, etc., and may be in unit-dosage forms suitable for administration of relatively precise dosages.

In some embodiments, the pharmaceutical composition may be administered by any suitable route, preferably in the form of a pharmaceutical composition adapted to such a route, and in a dose effective for the treatment intended. The carrier mixture may be filled into a gelatin capsule or compressed as a tablet. The pharmaceutical composition may be administered, in some embodiments, as an oral dosage form or as an infusion.

In some embodiments, the pharmaceutical composition is formulated for administration by inhalation. In some embodiments, the pharmaceutical composition is formulated for intrapulmonary administration. In some embodiments, the composition is a pharmaceutical composition.

In some embodiments, the pharmaceutical composition is formulated for oral administration. In some embodiments, the pharmaceutical composition is formulated for nasal administration. In some embodiments, the pharmaceutical composition is formulated for intranasal administration.

In some embodiments, pulmonary delivery is via nebulization of the compound using a nebulizer, preferably a mesh nebulizer. In some embodiments, the nebulizer delivers the compound to lung cells in the form of an aerosol.

In some embodiments, the pharmaceutical composition is formulated for abdominal administration. In some embodiments, the pharmaceutical composition is formulated for subcutaneous administration. In some embodiments, the pharmaceutical composition is formulated for intra-peritoneal administration. In some embodiments, the pharmaceutical composition is formulated for intravenous administration. In some embodiments, the pharmaceutical composition is formulated for intramuscular administration. In some embodiments, the pharmaceutical composition is formulated for mucosal administration. In some embodiments, parenteral administration comprises intravascular, intraperitoneal, subcutaneous, intramuscular, and intra sternal administration.

Any pharmaceutical composition contemplated herein can, for example, be delivered orally via any acceptable and suitable oral preparations. Exemplary oral preparations, include, but are not limited to, for example, tablets, troches, lozenges, aqueous and oily suspensions, dispersible powders or granules, emulsions, hard and soft capsules, liquid capsules, syrups, and elixirs.

Exemplary injectable forms include, but are not limited to, for example, sterile aqueous solutions comprising acceptable vehicles and solvents, such as, for example, water, Ringer's solution, and isotonic sodium chloride solution; sterile oil-in-water microemulsions; and aqueous or oleaginous suspensions.

In other embodiments, the pharmaceutical composition is formulated as a nanoparticle, lipid nanoparticle, microparticle or liposome. In one embodiment, the antisense oligonucleotide as described herein is within or attached to a nanoparticle, lipid nanoparticle, microparticle, or liposome.

In some embodiments, the pharmaceutical composition is formulated for systemic administration.

In some embodiments, the pharmaceutical composition comprises an antisense oligonucleotide as described herein. In some embodiments, the composition comprises at least 1 nM, at least 2.5 nM, at least 10 nM, at least 100 nM, at least 0.5 µM, at least 1 µM, at least 10 µM, at least 20 µM, at least 50 µM, at least 100 µM, at least 250 µM, or at least 500 µM of the antisense oligonucleotide or any value and range therebetween. In some embodiments, the composition comprises 2.5 nM to 10 nM, 1 nM to 100 nM, 1 nM to 0.5 µM, 1 nM to 1 µM, 1 nM to 10 µM, 10 nM to 20 µM, 100 nM to 50 µM, 1 µM to 100 µM, 1 µM to 250 µM, or1 µM to 500 µM, of the antisense oligonucleotide. In other embodiments, the composition comprises 5 nM to 25 nM, 10 nM to 50 nM, 20 nM to 100 nM, 50 nM to 0.5 µM of the antisense oligonucleotide. Each possibility represents a separate embodiment of the invention.

The pharmaceutically active compounds of this invention can be processed in accordance with conventional methods of pharmacy to produce medicinal agents for administration to subjects, including humans and other mammals. The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such pharmaceutical compositions may also comprise adjuvants, such as wetting, sweetening, flavoring, and perfuming agents.

The amounts of compounds that are administered and the dosage regimen for treating a disease or condition of this invention depends on a variety of factors, including the age, weight, gender, the medical condition of the subject, the type of disease, the severity of the disease, the route and frequency of administration, and the particular compound employed. Thus, the dosage regimen may vary widely, but can be determined routinely using standard methods.

For therapeutic purposes, the active compounds of this invention are ordinarily combined with one or more adjuvants appropriate to the indicated route of administration. If administered orally, the compounds may be admixed with lactose, sucrose, trehalose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

### Combination Treatments/Therapies

In some embodiments, compositions as described herein comprise an oligonucleotide as described herein and one or more active pharmaceutical ingredients for the treatment of a lung disease or disorder.

In some embodiments, the additional active pharmaceutical ingredient comprises a corticosteroid, a short-acting bronchodilator (e.g., albuterol, levalbuterol, ipratropium, or a combination of albuterol and ipratropium), a long-acting bronchodilator (e.g., aclidinium, arformoterol, formoterol, glycopyrrolate, indacaterol, olodaterol, salmeterol, tiotropium, umeclidinium or combinations thereof), methylxanthines and corticosteroids (e.g., fluticasone, budesonide, or prednisolone, alone or in combination with long-acting bronchodilator), or any combination thereof. In one embodiment, a long-acting bronchodilator comprises a long-acting beta agonist (LABA), a long-acting muscarinic antagonist (LAMA), or a combination thereof. In some embodiments, the additional active pharmaceutical ingredient comprises oral phosphodiesterase 4 inhibitors (e.g., roflumilast), macrolide antibiotics, or a combination thereof. In some embodiments, the latter ingredient is administered in addition to other active pharmaceutical ingredients and in addition to the antisense oligonucleotide described herein.

In other embodiments, the additional active pharmaceutical ingredient comprises an agent targeting mucus hypersecretion.

In one embodiment, the mucus-hypersecreting-targeting agent prevents cholinergic bronchoconstriction, which in one embodiment, is via high-affinity binding to transmembrane Gq-coupled airway muscarinic M3 receptors, which in one embodiment, inhibits ACh-dependent and IP3-mediated release of Ca2+ from intracellular stores. In some embodiments, the agent comprises Tiotropium.

In another embodiment, the mucus-hypersecreting-targeting agent comprises Roflumilast, which, in one embodiment, affects inflammation produced by eosinophils and neutrophils, airway remodeling, and bronchoconstriction.

In another embodiment, the mucus-hypersecreting-targeting agent binds cytokines and/or chemokines, such as IFN-γ and IL-8, protecting them from proteolytic degradation thus increasing their activities or binds effector molecules responsible for leukocyte recruitment to the site of infection/injury. In some embodiments, the agent comprises heparan sulfate.

In another embodiment, the mucus-hypersecreting-targeting agent decreases oxidative biomarkers in peripheral blood and exhaled breath condensate. In some embodiments, the agent comprises N-acetylcysteine.

In another embodiment, the mucus-hypersecreting-targeting agent comprises carbocisteine or another drug that is mucoactive with *in vitro* free-radical scavenging and anti-inflammatory properties.

In another embodiment, the mucus-hypersecreting-targeting agent comprises Salmeterol or Formoterol or another beta-2 adrenergic agonist.

In some embodiments, the corticosteroid is an inhaled corticosteroid (ICS). In other embodiments, the corticosteroid is a topical corticosteroid. In other embodiments, the additional active pharmaceutical ingredient comprises a LAMA, a LABA, and an ICS (triple inhaled therapy).

Accordingly, the combination therapies of the present invention may be administered together with other therapies useful in the treatment of a lung disease or disorder or related diseases. The invention herein further comprises use of a first composition and a second composition as described herein in preparing medicaments for the treatment of a lung disease or disorder or related diseases.

In other embodiments, the antisense oligonucleotides of the present invention can be formulated or co-administered with other therapeutic agents that are selected for their particular usefulness in addressing side effects associated with the conditions associated with a lung disease or disorder. For example, compounds of the invention may be formulated with agents to treat or suppress infections, such as antibiotics. In other embodiments, other therapeutic agents comprise aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indo-methicin, a barbiturate, a cephalosporin, a sulfa drug, an antidiabetic, or a combination thereof. In other embodiments, one or more therapeutic agents comprises a mucolytic agent, a bronchodialator, an antibiotic, an anti-infective agent, an anti-inflammatory agent, a corticosteroid, or a combination thereof.

The above other therapeutic agents, when employed in combination with the compounds of the present invention, may be used, for example, in those amounts indicated in the Physicians' Desk Reference (PDR) or as otherwise determined by one of ordinary skill in the art.

### Methods of Use

In some embodiments, the present invention provides a method for treating, preventing, ameliorating, or slowing progression of a lung disease or disorder in a subject.

In some embodiments, the subject has a lung disease or disorder. In some embodiments, the individual is at risk for developing a lung disease or disorder. In some embodiments, the lung disease or disorder is associated with overexpression of MUC5AC, MUC5B, or a combination thereof. In some embodiments, the subject has a lung disease or disorder that is not a cancer or tumor or neoplasm.

In other embodiments, the lung disease or disorder is associated with normal expression of MUC5AC, MUC5B, or a combination thereof. In some embodiments, normal expression is within the range of expression measured in healthy subjects.

In some embodiments, a subject as described herein has over-expression of MUC5AC, MUC5B, or both MUC5AC and MUC5B mRNA. In other embodiments, the subject has normal expression of MUC5AC, MUC5B, or both MUC5AC and MUC5B mRNA. In some embodiments, a subject as described herein has over-expression of MUC5AC, MUC5B, or both MUC5AC and MUC5B protein. In other embodiments, subject has normal expression of MUC5AC, MUC5B, or both MUC5AC and MUC5B protein. In some embodiments, over-expression of mRNA or protein is relative to healthy subjects.

In some embodiments, over-expression as described herein comprises a 15%-95% increase compared with control tissue. In other embodiments, over-expression comprises a 15% increase; a 20% increase; a 25% increase; a 30% increase; a 35% increase; increase; a 40% increase; a 45% increase; a 50% increase; a 55% increase; a 60% increase; a 65% increase; a 70% increase; a 75% increase; a 80% increase; a 85% increase; a 90% increase; or a 95% increase compared with control tissue. In other embodiments, over-expression comprises a 150% increase; a 200% increase; a 250% increase; a 300% increase; a 400% increase; a 450% increase; a 500% increase compared with control tissue.

In some embodiments, mRNA or protein level is compared to control tissue from the subject being treated prior to the onset of symptoms. In other embodiments, control tissue is from one or more subjects not having a lung disease or disorder as described herein. In other embodiments, control tissue is from one or more subjects not being diagnosed with a lung disease or disorder as described herein.

In some embodiments, a subject as described herein has increased activation of MUC5AC, MUC5B, or both MUC5AC and MUC5B protein. In some embodiments, the subject has increased secretion of MUC5AC, MUC5B, or both MUC5AC and MUC5B protein.

In some embodiments, increased activation and increased secretion as described herein comprises a 15%-95% increase compared with control tissue. In other embodiments, over-expression comprises a 15% increase; a 20% increase; a 25% increase; a 30% increase; a 35% increase; increase; a 40% increase; a 45% increase; a 50% increase; a 55% increase; a 60% increase; a 65% increase; a 70% increase; a 75% increase; a 80% increase; a 85% increase; a 90% increase; or a 95% increase compared with control tissue. In other embodiments, over-expression comprises a 150% increase; a 200% increase; a 250% increase; a 300% increase; a 400% increase; a 450% increase; a 500% increase compared with control tissue.

In other embodiments, a subject as described herein has normal activation of MUC5AC, MUC5B, or both MUC5AC and MUC5B protein. In some embodiments, the subject has normal secretion of MUC5AC, MUC5B, or both MUC5AC and MUC5B protein.

In some embodiments, the subject has reduced mucociliary function, reduced alveolar repair, and/or increased lung fibrosis.

In some embodiments, the present invention provides a method for treating a lung disease or disorder in a subject, comprising the step of administering to said subject an oligonucleotide as described herein. In other embodiments, the present invention provides a method for treating a lung disease or disorder in a subject, comprising the step of administering to said subject a vector as described herein. In other embodiments, the present invention provides a method for treating a lung disease or disorder in a subject, comprising the step of administering to said subject a cell as described herein. In other embodiments, the present invention provides a method for treating a lung disease or disorder in a subject, comprising the step of administering to said subject a composition as described herein.

In some embodiments, the lung disease or disorder comprises an airway disease. In some embodiments, the lung disease or disorder comprises a hypersecretory respiratory disease. In some embodiments, the lung disease or disorder comprises a muco-obstructive disease.

In one embodiment, the muco-obstructive disease comprises asthma. In another embodiment, the muco-obstructive disease comprises bronchitis. In another embodiment, the muco-obstructive disease comprises chronic obstructive pulmonary disease (COPD). In another embodiment, the muco-obstructive disease comprises cystic fibrosis (CF). In another embodiment, the muco-obstructive disease comprises non-CF Bronchiectasis (NCFB). In another embodiment, the muco-obstructive disease comprises primary ciliary dyskinesia. In another embodiment, the muco-obstructive disease comprises bronchiolitis obliterans. In another embodiment, the muco-obstructive disease comprises a combination of any two or more of the muco-obstructive diseases described herein.

In some embodiments, the subject has epithelial defects in ion-fluid transport, excessive mucus secretion, or a combination thereof. In some embodiments, the subject has hyperconcentrated or dehydrated mucus, failed mucus transport, mucus adhesion to airway surfaces, or a combination thereof. In other embodiments, the subject has normal levels of mucus secretion. In some embodiments, there is a change in the ratio between water and soluble components or solid materials within the mucus.

In some embodiments, the lung disease or disorder comprises a pulmonary fibrosis or a lung fibrosis. In one embodiment, the pulmonary fibrosis comprises idiopathic pulmonary fibrosis (IPF). In another embodiment, the pulmonary fibrosis comprises progressive pulmonary fibrosis (PPF). In another embodiment, the pulmonary fibrosis comprises fibrotic hypersensitivity pneumonitis. In another embodiment, the pulmonary fibrosis comprises connective tissue disease-associated interstitial lung disease (CTD-ILD). In some embodiments, the CTD-ILD comprises scleroderma-ILD, systemic sclerosis-ILD, Rheumatoid arthritis (RA)-ILD, Sjogren-ILD, systemic lupus erythematosus-ILD, polymyositis-ILD, dermatomyositis-ILD, mixed connective tissue disease-ILD, undifferentiated connective tissue disease-ILD, or another autoimmune-ILD known in the art.

In other embodiments, the lung disease or disorder comprises asthma, chronic rhinosinusitis (CRS), chronic obstructive pulmonary disease (COPD), diffuse panbronchiolitis (DPB), bronchitis, bronchiolitis obliterans, bronchiectasis, Non Cystic Fibrosis Bronchiectasis (NCFB), Primary Ciliary Dyskinesia (PCD), or bronchiectasis. In another embodiment, the pulmonary fibrosis comprises cystic fibrosis (CF). In other embodiments, the lung disease or disorder comprises lung cancer. In other embodiments, the lung disease or disorder comprises pneumonia, pulmonary emphysema, pulmonary edema, pulmonary embolus, pulmonary hypertension, sarcoidosis, pneumothorax, atelectasis, or a combination thereof. In other embodiments, the lung disease or disorder comprises idiopathic pulmonary fibrosis (IPF), progressive pulmonary fibrosis (PPF), fibrotic hypersensitivity pneumonitis, or CTD-ILD.

In some embodiments, asthma comprises allergic asthma, exercise-induced bronchoconstriction, cough-variant asthma, occupational asthma, nighttime (nocturnal) asthma, non-allergic asthma, or a combination thereof.

In some embodiments, the present invention provides a method for treating a major pulmonary fibrosis disease in a subject, comprising the step of administering to said subject an oligonucleotide as described herein. In other embodiments, the present invention provides a method for treating a major pulmonary fibrosis disease in a subject, comprising the step of administering to said subject a vector as described herein. In other embodiments, the present invention provides a method for treating a major pulmonary fibrosis disease in a subject, comprising the step of administering to said subject a cell as described herein. In other embodiments, the present invention provides a method for treating a major pulmonary fibrosis disease in a subject, comprising the step of administering to said subject a composition as described herein.

In some embodiments, the major pulmonary fibrosis disease comprises cystic fibrosis. In other embodiments, the major pulmonary fibrosis disease comprises idiopathic pulmonary fibrosis. In some embodiments, idiopathic pulmonary fibrosis is characterized by a chronic progressive decline in lung function, scarring of lung tissue, dyspnea (shortness of breath), or a combination thereof.

In some embodiments, the present invention provides a method for treating cystic fibrosis in a subject, comprising the step of administering to said subject an oligonucleotide, composition, vector, or cell as described herein. In some embodiments, the present invention provides a method for suppressing cystic fibrosis in a subject, comprising the step of administering to said subject an oligonucleotide, composition, vector, or cell as described herein. In some embodiments, the present invention provides a method for inhibiting cystic fibrosis in a subject, comprising the step of administering to said subject an oligonucleotide, composition, vector, or cell as described herein.

In some embodiments, the present invention provides a method for treating idiopathic pulmonary fibrosis in a subject, comprising the step of administering to said subject an oligonucleotide, composition, vector, or cell as described herein. In some embodiments, the present invention provides a method for suppressing idiopathic pulmonary fibrosis in a subject, comprising the step of administering to said subject an oligonucleotide, composition, vector, or cell as described herein. In some embodiments, the present invention provides a method for inhibiting idiopathic pulmonary fibrosis in a subject, comprising the step of administering to said subject an oligonucleotide, composition, vector, or cell as described herein.

In some embodiments, the present invention provides a method for treating CRS in a subject, comprising the step of administering to said subject an oligonucleotide, composition, vector, or cell as described herein. In some embodiments, the present invention provides a method for suppressing CRS in a subject, comprising the step of administering to said subject an oligonucleotide, composition, vector, or cell as described herein. In some embodiments, the present invention provides a method for inhibiting CRS in a subject, comprising the step of administering to said subject an oligonucleotide, composition, vector, or cell as described herein.

In some embodiments, the present invention provides a method for treating COPD in a subject, comprising the step of administering to said subject an oligonucleotide, composition, vector, or cell as described herein. In some embodiments, the present invention provides a method for suppressing COPD in a subject, comprising the step of administering to said subject an oligonucleotide, composition, vector, or cell as described herein. In some embodiments, the present invention provides a method for inhibiting COPD in a subject, comprising the step of administering to said subject an oligonucleotide, composition, vector, or cell as described herein.

In some embodiments, the present invention provides a method for treating DPB in a subject, comprising the step of administering to said subject an oligonucleotide, composition, vector, or cell as described herein. In some embodiments, the present invention provides a method for suppressing DPB in a subject, comprising the step of administering to said subject an oligonucleotide, composition, vector, or cell as described herein. In some embodiments, the present invention provides a method for inhibiting DPB in a subject, comprising the step of administering to said subject an oligonucleotide, composition, vector, or cell as described herein.

In some embodiments, the present invention provides a method for treating asthma in a subject, comprising the step of administering to said subject an oligonucleotide, composition, vector, or cell as described herein. In some embodiments, the present invention provides a method for suppressing asthma in a subject, comprising the step of administering to said subject an oligonucleotide, composition, vector, or cell as described herein. In some embodiments, the present invention provides a method for inhibiting asthma in a subject, comprising the step of administering to said subject an oligonucleotide, composition, vector, or cell as described herein.

In some embodiments, the present invention provides a method for treating Primary Ciliary Dyskinesia in a subject, comprising the step of administering to said subject an oligonucleotide, composition, vector, or cell as described herein. In some embodiments, the present invention provides a method for suppressing Primary Ciliary Dyskinesia in a subject, comprising the step of administering to said subject an oligonucleotide, composition, vector, or cell as described herein. In some embodiments, the present invention provides a method for inhibiting Primary Ciliary Dyskinesia in a subject, comprising the step of administering to said subject an oligonucleotide, composition, vector, or cell as described herein.

In some embodiments, the present invention provides a method for treating bronchiectasis in a subject, comprising the step of administering to said subject an oligonucleotide, composition, vector, or cell as described herein. In some embodiments, the present invention provides a method for suppressing bronchiectasis in a subject, comprising the step of administering to said subject an oligonucleotide, composition, vector, or cell as described herein. In some embodiments, the present invention provides a method for inhibiting bronchiectasis in a subject, comprising the step of administering to said subject an oligonucleotide, composition, vector, or cell as described herein.

In some embodiments, the present invention provides a method for treating NCFB in a subject, comprising the step of administering to said subject an oligonucleotide, composition, vector, or cell as described herein. In some embodiments, the present invention provides a method for suppressing NCFB in a subject, comprising the step of administering to said subject an oligonucleotide, composition, vector, or cell as described herein. In some embodiments, the present invention provides a method for inhibiting NCFB in a subject, comprising the step of administering to said subject an oligonucleotide, composition, vector, or cell as described herein.

In some embodiments, the present invention provides a method for treating bronchiolitis obliterans in a subject, comprising the step of administering to said subject an oligonucleotide, composition, vector, or cell as described herein. In some embodiments, the present invention provides a method for suppressing bronchiolitis obliterans in a subject, comprising the step of administering to said subject an oligonucleotide, composition, vector, or cell as described herein. In some embodiments, the present invention provides a method for inhibiting bronchiolitis obliterans in a subject, comprising the step of administering to said subject an oligonucleotide, composition, vector, or cell as described herein.

In some embodiments, the present invention provides a method for inhibiting mucous formation or excessive mucus formation in a subject, comprising the step of administering to said subject an oligonucleotide, composition, vector, or cell as described herein. In one embodiment, overexpression of MUC5AC, MUC5B, or both leads to excessive mucus formation. In some embodiments, mucus overexpression causes or exacerbates the symptoms associated with common lung diseases or disorders. In some embodiments, the present invention provides a method for increasing mucociliary function, increasing alveolar repair, decreasing lung fibrosis, or a combination thereof.

In some embodiments, a subject as described herein has a polymorphism in the promoter region of MUC5B. In one embodiment, the polymorphism comprises rs35705950, which, in some embodiments, is associated with familial and sporadic forms of idiopathic pulmonary fibrosis. In some embodiments, overexpression of MUC5B can result in one or more of the following outcomes: reduced mucociliary function, reduced alveolar repair, and increased lung fibrosis.

In other embodiments, the subject has a polymorphism in MUC5AC. In one embodiment, the polymorphism comprises rs1132440, rs17859812, or both. In other embodiments, the polymorphism comprises the MUC5AC 6.4 kb variable number tandem repeat (VNTR) allele.

Thus, in some embodiments, the lung disease or disorder to be treated using the methods described herein is the result of a genetic mutation or mutations or a genetic predisposition to the lung disease or disorder. In other embodiments, the lung disease or disorder is the result of workplace air pollutants, which in one embodiment, comprise organic dust, inorganic dust, chemical products, fumes, or a combination thereof. In other embodiments, the lung disease or disorder results from household air pollution, which in one embodiment, comprises chemical fire, coal fire, poor ventilation, or a combination thereof. In other embodiments, the lung disease or disorder results from smoke, which in one embodiment, comprises tobacco smoke. In some embodiments, the smoke exposure is at prenatal or postnatal stages, while, in other embodiments, the smoke exposure is in adulthood. In some embodiments, the smoke exposure is direct, while in other embodiments, the smoke exposure is indirect or "second-hand."

In some embodiments, the subject for treatment using the methods as described herein comprise subjects having mucus metaplasia, bronchiolar smooth-muscle hypertrophy, mural edema, peribronchiolar fibrosis, excess mucus production in small airways, or a combination thereof.

Accordingly, in some embodiments, the methods of the present invention further comprise identifying a subject having a polymorphism prior to the step of treating the subject with one or more of the antisense oligonucleotides as described herein.

In some embodiments, the present invention provides a method for reducing the level of MUC5AC, MUC5B, or both MUC5AC and MUC5B mRNA and/or protein in a subject. In some embodiments, the oligonucleotide achieves at least 25% reduction in MUC5AC, MUC5B, or both MUC5AC and MUC5B mRNA and/or protein levels. In some embodiments, the oligonucleotide achieves at least 30% reduction in MUC5AC, MUC5B, or both MUC5AC and MUC5B mRNA and/or protein levels. In some embodiments, the oligonucleotide achieves at least 40% reduction in MUC5AC, MUC5B, or both MUC5AC and MUC5B mRNA and/or protein levels. In some embodiments, the oligonucleotide achieves at least 50% reduction in MUC5AC, MUC5B, or both MUC5AC and MUC5B mRNA and/or protein levels. In some embodiments, the oligonucleotide achieves at least 60% reduction in MUC5AC, MUC5B, or both MUC5AC and MUC5B mRNA and/or protein levels. In some embodiments, the oligonucleotide achieves at least 70% reduction in MUC5AC, MUC5B, or both MUC5AC and MUC5B mRNA and/or protein levels. In some embodiments, the oligonucleotide achieves at least 80% reduction in MUC5AC, MUC5B, or both MUC5AC and MUC5B mRNA and/or protein levels. In some embodiments, the oligonucleotide achieves at least 85% reduction in MUC5AC, MUC5B, or both MUC5AC and MUC5B mRNA and/or protein levels. In some embodiments, the oligonucleotide achieves at least 90% reduction in MUC5AC, MUC5B, or both MUC5AC and MUC5B mRNA and/or protein levels. In some embodiments, the oligonucleotide achieves at least 95% reduction in MUC5B mRNA and/or protein levels.

In some embodiments, the oligonucleotide achieves at least 10-90% reduction in MUC5AC, MUC5B, or both MUC5AC and MUC5B mRNA and/or protein levels. In some embodiments, the oligonucleotide achieves at least 20-80% reduction in MUC5AC, MUC5B, or both MUC5AC and MUC5B mRNA and/or protein levels. In some embodiments, the oligonucleotide achieves at least 30-70% reduction in MUC5AC, MUC5B, or both MUC5AC and MUC5B mRNA and/or protein levels. In some embodiments, the oligonucleotide achieves at least 40-60% reduction in MUC5AC, MUC5B, or both MUC5AC and MUC5B mRNA and/or protein levels. In some embodiments, the oligonucleotide achieves at least 30-40% reduction in MUC5AC, MUC5B, or both MUC5AC and MUC5B mRNA and/or protein levels.

In some embodiments, the present invention provides a method for reducing the level of functional or non-functional or both functional and non-functional MUC5AC, MUC5B, or both MUC5AC and MUC5B mRNA.

In other embodiments, the present invention provides a method of modulating splicing or expression of a MUC5AC, MUC5B, or both a MUC5AC and MUC5B transcript in a cell comprising contacting the cell with an antisense oligonucleotide, a vector, a cell, or a composition as described herein.

In some embodiments, "modulating" as used herein means changing the amount or quality of a molecule, function, or activity when compared to the amount or quality of a molecule, function, or activity prior to modulation. For example, modulation includes a change or a decrease (inhibition or reduction) in gene expression. As a further example, modulation of expression can include a change in splice site selection of pre-mRNA processing, resulting in a change in the absolute or relative amount of a particular splice-variant compared to the amount in the absence of modulation.

In other embodiments, the present invention provides a method of prolonging survival in a subject having a lung disease or disorder as described herein, comprising the steps of administering to the subject one or more antisense oligonucleotides as described herein. In other embodiments, the present invention provides a method of delaying progression of a lung disease or disorder as described herein in a subject having a lung disease or disorder as described herein, comprising the steps of administering to the subject one or more antisense oligonucleotides as described herein. In other embodiments, the present invention provides a method of avoiding resistance to therapy in a subject having a lung disease or disorder as described herein, comprising the steps of administering to the subject one or more antisense oligonucleotides as described herein.

In some embodiments, the present invention provides the use of an antisense oligonucleotide as described herein, or a pharmaceutical composition comprising the antisense oligonucleotide, for treating, suppressing or inhibiting a lung disease or disorder in a subject. In some embodiments, an antisense oligonucleotide as described herein, or a pharmaceutical composition comprising the antisense oligonucleotide, is used for improving at least one clinical parameter of a lung disease or disorder in a subject. In some embodiments, an antisense oligonucleotide as described herein, or a pharmaceutical composition comprising the antisense oligonucleotide, is used for alleviating symptoms of a lung disease or disorder in a subject.

In some embodiments, the present invention provides the use of a therapeutically acceptable amount of the compositions as described herein for treating, suppressing or inhibiting a lung disease or disorder in a subject. In other embodiments, the present invention provides the use of a therapeutically effective amount of the compositions as described herein for treating, suppressing or inhibiting a lung disease or disorder in a subject. In other embodiments, the present invention provides the use of a synergistically effective amount of a combination therapy as described herein for treating, suppressing or inhibiting a lung disease or disorder in a subject.

In some embodiments, the methods of the present invention further comprise the step of identifying a candidate subject for treatment with the compositions as described herein comprising the step of determining the levels of MUC5AC, MUC5B, or both MUC5AC and MUC5B mRNA in the subject. In other embodiments, the methods of the present invention further comprise the step of identifying a candidate subject for treatment with the compositions as described herein comprising the step of determining the levels of MUC5AC, MUC5B, or both MUC5AC and MUC5B protein in the subject. In other embodiments, the methods of the present invention further comprise the step of identifying a candidate subject for treatment with the compositions as described herein comprising the step of determining the functionality of MUC5AC, MUC5B, or both MUC5AC and MUC5B in the subject.

In other embodiments, the methods of the present invention further comprise the step of identifying a candidate subject for treatment with the compositions as described herein comprising the step of evaluating MUC5AC, MUC5B, or both MUC5AC and MUC5B gene function in the subject. In some embodiments, evaluating MUC5AC, MUC5B, or both MUC5AC and MUC5B gene function comprises determining if there are MUC5AC, MUC5B, or both MUC5AC and MUC5B mutations and if so, where the MUC5AC, MUC5B, or both MUC5AC and MUC5B mutations are located.

In some embodiments, evaluating MUC5AC, MUC5B, or both MUC5AC and MUC5B gene function comprises RNA-seq or another RNA sequencing tool to reveal the presence and quantity of RNA in a biological sample at a given point in time. In other embodiments, other methods of evaluating the quantity of MUC5AC, MUC5B, or both MUC5AC and MUC5B RNA that are known in the art may be utilized.

In other embodiments, the present invention provides a method of treating a lung disease or disorder comprising the step of evaluating the genetic material in a biological sample from the subject. In one embodiment, the genetic material is evaluated using an RNA sequencing method. In another embodiment, the genetic material is evaluated using a DNA sequencing method. In another embodiment, protein levels and/or function are evaluated in a biological sample from the subject.

In some embodiments, the biological sample comprises tissue. In some embodiments, the biological sample comprises blood cells. In other embodiments, the biological sample comprises a sputum sample. In other embodiments, the biological sample comprises epithelial cells, which, in some embodiments, are sampled from the inside of the subject's mouth. In other embodiments, the biological sample comprises nasal cells. In other embodiments, the biological sample comprises lung cells. It is to be understood that in some embodiments, more than one biological sample may be collected from the subject.

In some embodiments, the present invention provides a method for improving at least one clinical parameter of a lung disease or disorder in a subject. In other embodiments, the present invention provides a method for alleviating symptoms of a lung disease or disorder in a subject.

In some embodiments, alleviation of symptoms as described herein comprises a measured improvement of 15%-95%. In other embodiments, the measured improvement comprises a 15% improvement; a 20% improvement; a 25% improvement; a 30% improvement; a 35% improvement; improvement; a 40% improvement; a 45% improvement; a 50% improvement; a 55% improvement; a 60% improvement; a 65% improvement; a 70% improvement; a 75% improvement; a 80% improvement; a 85% improvement; a 90% improvement; or a 95% improvement.

In some embodiments, the methods as described herein comprise the step of administering a splicing modulator, which, in some embodiments, is an antisense oligonucleotide, which, in some embodiments, is a synthetic antisense oligonucleotide.

In other embodiments, the methods as described herein comprise the step of contacting a cell with a splicing modulator, which, in some embodiments, is an antisense oligonucleotide, which, in some embodiments, is a synthetic antisense oligonucleotide. In some embodiments, contacting comprises *in vivo, in vitro,* or *ex vivo* contacting.

In some embodiments, the subject has at least one mutation in a MUC5AC gene, MUC5B gene, or in both the MUC5AC and MUC5B genes or in the mRNA transcribed therefrom. In some embodiments, the subject is homozygous for one or more MUC5AC and/or MUC5B mutations. In some embodiments, the subject is heterozygous for one or more MUC5AC and/or MUC5B mutations.

In other embodiments, the subject has at least one polymorphism in a MUC5AC gene, MUC5B gene, or in both the MUC5AC and MUC5B genes or in the mRNA transcribed therefrom. In some embodiments, the subject is homozygous for one or more MUC5AC and/or MUC5B polymorphisms. In some embodiments, the subject is heterozygous for one or more MUC5AC and/or MUC5B polymorphisms. In some embodiment, the polymorphism is a single nucleotide polymorphism (SNP). In other embodiments, the polymorphism is a small-scale insertion/deletion. In other embodiments, the polymorphism is a polymorphic repetitive element. In other embodiments, the polymorphism is a micro satellite variation.

In some embodiments, the subject is suffering from lung inflammation. In some embodiments, the lung cells of the subject are overactivated. According to this aspect and in some embodiments, the lung cells are hyper-proliferative. In other embodiments, the lung cells produce and/or secrete abnormal levels of mucins. In some embodiments, the subject has hyperplasia of goblet cells.

In some embodiments, at least some of the cells of the subject over-express one or more mucins. In some embodiments, at least some of the cells of the subject over-express MUC5AC, MUC5B, or both MUC5AC and MUC5B mRNA. In some embodiments, at least some of the cells of the subject over-express MUC5AC, MUC5B, or both MUC5AC and MUC5B protein. In some embodiments, MUC5AC, MUC5B, or both MUC5AC and MUC5B in the airway cells of said subject are hyper-functional or hyperactive.

In some embodiments, the MUC5AC and/or MUC5B protein in the cells of the subject comprise a mixture of wild type, full-length, and fully functional MUC5AC and/or MUC5B protein encoded by the wild type allele and a mutated and deleterious MUC5AC and/or MUC5B protein encoded by the mutated allele.

In some embodiments, "a mutation" as described herein refers to one or more nucleotide substitutions or modifications which render a partially or fully non-functional protein. In some embodiments, a modification comprises an insertion, deletion, inversion, or a combination thereof. In some embodiment, the modification results in a disease phenotype in a subject harboring or comprising the modification.

In other embodiments, the present invention provides a method of treating, suppressing, or inhibiting a lung disease or disorder in a subject comprising the step of administering to the subject a composition comprising one or more antisense oligonucleotides as described herein. In other embodiments, the present invention provides a method of treating, suppressing, or inhibiting a lung disease or disorder in a subject comprising the step of administering to the subject a composition consisting essentially of one or more antisense oligonucleotides as described herein. In other embodiments, the present invention provides a method of treating, suppressing, or inhibiting a lung disease or disorder in a subject comprising the step of administering to the subject a composition consisting of one or more antisense oligonucleotides as described herein.

In other embodiments, the present invention provides a method of treating, suppressing, or inhibiting a cancer in a subject comprising the step of administering to the subject a composition comprising one or more antisense oligonucleotides as described herein. In other embodiments, the present invention provides a method of treating, suppressing, or inhibiting a cancer in a subject comprising the step of administering to the subject a composition consisting essentially of one or more antisense oligonucleotides as described herein. In other embodiments, the present invention provides a method of treating, suppressing, or inhibiting a cancer in a subject comprising the step of administering to the subject a composition consisting of one or more antisense oligonucleotides as described herein. In some embodiments, a symptom of the cancer is increased mucin production. In some embodiments, the cancer comprises pancreas, lung, breast, ovary, or colon cancer.

In other embodiments, the lung disease or disorder does not comprise lung cancer. In other embodiments, the lung disease or disorder does not comprise a lung adenocarcinoma. In other embodiments, the lung disease or disorder does not comprise a non-small cell lung cancer. In other embodiments, the lung disease or disorder comprises a cancer that does not comprise a KRAS mutation.

In other embodiments, the present invention provides a method of treating a non-cancerous lung disease or disorder in a subject, comprising the step of administering to said subject a composition comprising a synthetic antisense oligonucleotide targeting MUC5AC, MUC5B, or both MUC5AC and MUC5B sequences. In other embodiments, the present invention provides a method of suppressing or inhibiting a non-cancerous lung disease or disorder in a subject, comprising the step of administering to said subject a composition comprising a synthetic antisense oligonucleotide targeting MUC5AC, MUC5B, or both MUC5AC and MUC5B sequences.

In other embodiments, the present invention provides a method of reducing the levels of MUC5AC, MUC5B, or both MUC5AC and MUC5B in a cell of a subject comprising the step of administering to said subject an oligonucleotide, vector, cell, or composition as described herein. In other embodiments, the present invention provides a method of modulating MUC5AC or MUC5B expression or production. In some embodiments, MUC5A and MUC5B expression or production are modulated independently. In other embodiments, both MUC5A and MUC5B expression or production are modulated together.

In other embodiments, the present invention provides a method of inducing nonsense-mediated decay of MUC5AC, MUC5B, or both MUC5AC and MUC5B in a cell of a subject comprising the step of administering to said subject an oligonucleotide, vector, cell, or composition as described herein. In one embodiment, the oligonucleotide does not activate the RNAse H pathway.

In other embodiments, the present invention provides a method of splice switching MUC5AC, MUC5B, or both MUC5AC and MUC5B in a cell of a subject comprising the step of administering to said subject an oligonucleotide, vector, cell, or composition as described herein.

In other embodiments, the present invention provides a method of introducing an early termination codon to a MUC5AC, MUC5B, or both MUC5AC and MUC5B mRNA in a cell of a subject comprising the step of administering to said subject an oligonucleotide, vector, cell, or composition as described herein.

In other embodiments, the present invention provides a method of skipping over an exon of MUC5AC, MUC5B, or both MUC5AC and MUC5B mRNA in a cell of a subject comprising the step of administering to said subject an oligonucleotide, vector, cell, or composition as described herein.

In other embodiments, the present invention provides a method of exon skipping MUC5AC, MUC5B, or both MUC5AC and MUC5B mRNA, which leads to the generation of a premature stop codon. In some embodiments, transcripts carrying the stop codon are subjected to degradation by the Nonsense Mediated mRNA Decay mechanism (NMD), leading to a reduction in the transcript level. In some embodiments, transcripts that escape the NMD are translated to a short and non-functional protein.

In other embodiments, the present invention provides a method of improving mucociliary clearance (MCC) in a subject. In other embodiments, the present invention provides a method of reducing mucin hyperconcentration. In other embodiments, the present invention provides a method of decreasing transient respiratory infection. In other embodiments, the present invention provides a method of decreasing respiratory disease in a subject or the pathogenesis of respiratory disease in the subject. In other embodiments, the present invention provides a method of altering the relative amounts of MUC5AC and MUC5B in a cell or tissue of a subject. In other embodiments, the present invention provides a method of decreasing mucin concentration in sputum in a subject with CF. In other embodiments, the present invention provides a method of decreasing partial osmotic pressure, osmotic compression of the periciliary layer, mucociliary stasis in a subject with CF.

In other embodiments, the present invention provides a method of reducing mucus accumulation in the airway of a subject. In other embodiments, the present invention provides a method of reducing obstruction, infection, inflammation in the airway of a subject.

In some embodiments, a composition comprising a synthetic antisense oligonucleotide targeting MUC5AC, MUC5B, or both MUC5AC and MUC5B sequences is administered to said subject.

### Timing and Site of Administration

In some embodiments, in the methods of the present invention, one or more compositions or the antisense oligonucleotides as described herein is administered to a subject once per day. In some embodiments, one or more compositions or the antisense oligonucleotides as described herein are administered to a subject twice per day. In some embodiments, one or more compositions or the antisense oligonucleotides as described herein are administered to a subject three times per day. In some embodiments, one or more compositions or antisense oligonucleotides as described herein are administered to a subject four times per day. In some embodiments, one or more compositions or antisense oligonucleotides as described herein are administered to a subject once per week. In some embodiments, in the methods of the present invention, one or more compositions or the antisense oligonucleotides as described herein are administered once every two weeks. In some embodiments, in the methods of the present invention, one or more compositions or the antisense oligonucleotides as described herein are administered once every three weeks.

In some embodiments, one or more compositions as described herein is administered to a subject in one to four doses per day. In other embodiments, one or more compositions or antisense oligonucleotides as described herein are administered to a subject once every two days, once every three days, or twice a week.

In some embodiments, in the methods of the present invention, a composition as described herein comprising one or more antisense oligonucleotides is administered intermittently. In some embodiments, the intermittent administration comprises two days on/five days off. In other embodiments, the intermittent administration comprises three days on/four days off.

In some embodiments, a composition as described herein is administered on at least one day, at least two days, at least three days, at least four days, at least five days, at least six days, at least seven days, at least eight days, at least nine days, at least ten days, at least eleven days, at least twelve days, at least 13 days, at least 14 days, at least 21 days, or all 28 days of a 28-day treatment cycle.

In some embodiments, one or more of the compositions as described herein is administered for 7 days to 28 days. In other embodiments, one or more of the compositions as described herein is administered for 7 days to 8 weeks. In other embodiments, one or more of the compositions as described herein is administered for 7 days to 50 days. In other embodiments, one or more of the compositions as described herein is administered for 7 days to six months. In other embodiments, one or more of the compositions as described herein is administered for 7 days to one and half years. In other embodiments, one or more of the compositions as described herein is administered for 14 days to 12 months. In other embodiments, one or more of the compositions as described herein is administered for 14 days to 3 years. In other embodiments, one or more of the compositions as described herein is administered for one month to six months. In other embodiments, one or more of the compositions as described herein is administered for several years.

In some embodiments, one or more of the compositions as described herein is administered for 7 days. In other embodiments, one or more of the compositions as described herein is administered for 14 days. In other embodiments, one or more of the compositions as described herein is administered for 21 days. In other embodiments, one or more of the compositions as described herein is administered for 28 days. In other embodiments, one or more of the compositions as described herein is administered for 50 days. In other embodiments, one or more of the compositions as described herein is administered for 56 days. In other embodiments, one or more of the compositions as described herein is administered for 84 days. In other embodiments, one or more of the compositions as described herein is administered for 90 days. In other embodiments, one or more of the compositions as described herein is administered for 120 days.

In some embodiments, in the methods of the present invention, a first and second composition are administered for the desired treatment. In some embodiments, in the methods of the present invention, the first composition and the second composition are administered concurrently, i.e. at the same approximate time. In some embodiments, the first and second compositions are administered at the same site of administration. In some embodiments, in the methods of the present invention, the first composition and the second composition are administered at separate sites or at separate times. In some embodiments, the first composition is administered prior to the second composition. In other embodiments, the first composition is administered after the second composition. In some embodiments, concurrent administration comprises administering a single composition comprising the second composition and the first composition. In other embodiments, concurrent administration comprises administering separate compositions at approximately the same time.

In some embodiments, the first composition is administered several days before and after the administration of the second composition. In some embodiments, the first composition is administered 1, 2, 3, 4, or 5 days prior to the administration of the second composition. In some embodiments, the first composition is administered 1, 2, 3, 4, or 5 days subsequent to the administration of the second composition.

In some embodiments, the compositions of the present invention are administered to the subject on the same days. In some embodiments, the compositions of the present invention are administered to the subject on the different days.

The number of times a first or second composition is administered to a subject in need thereof depends on the discretion of a medical professional, the disorder, the severity of the disorder, and the subject's response to the formulation. In some embodiments, the first and second compositions disclosed herein are administered once to a subject in need thereof with a mild acute condition. In some embodiments, the first and second compositions disclosed herein are administered more than once to a subject in need thereof with a moderate or severe acute condition. In the case wherein the subject's condition does not improve, upon the doctor's discretion the first or second composition may be administered chronically, that is, for an extended period of time, including throughout the duration of the subject's life in order to ameliorate or otherwise control or limit the symptoms of the subject's disease or condition.

In the case wherein the subject's status does improve, upon the doctor's discretion, the first or second composition may be administered continuously; or, the dose of drug being administered may be temporarily reduced or temporarily suspended for a certain length of time (i.e., a "drug holiday"). In some embodiments, the length of the drug holiday varies between 2 days and 1 year. The dose reduction during a drug holiday may be from 10%- 100%, including by way of example only 10%, 15%, 20%, 25%, 30%, 35%, 40%, 45%, 50%, 55%, 60%, 65%, 70%, 75%, 80%, 85%, 90%, 95%, and 100%.

In some embodiments, the antisense oligonucleotides are delivered to cells that produce mucins, which in one embodiment, comprise epithelial cells, goblet cells, submucosal glands, club (clara) cells, or a combination thereof.

In some embodiments, MUC5AC antisense oligonucleotides are targeted to goblet cells. In some embodiments, MUC5AC antisense oligonucleotides are targeted to proximal, cartilaginous airways. In some embodiments, MUC5AC antisense oligonucleotides are administered to subjects with asthma.

In some embodiments, MUC5B antisense oligonucleotides are targeted to submucosal gland. In some embodiments, MUC5B antisense oligonucleotides are targeted to distal airway superficial epithelium. In some embodiments, MUC5B antisense oligonucleotides are administered to subjects with COPD. In other embodiments, MUC5B antisense oligonucleotides are administered to subjects with IPF.

### Experimental Procedures

### In vivo testing of oligonucleotides

Oligonucleotides are tested in animals to assess their ability to reduce MUC5 mRNA and/or protein levels and produce phenotypic changes, such as improved mucociliary function, improved alveolar repair and reduced lung fibrosis. Testing may be performed in normal animals, or in experimental disease models. Calculation of oligonucleotide dosage and dosing frequency is within the abilities of those skilled in the art and depends upon factors such as route of administration and animal body weight. Following a period of treatment with oligonucleotides, RNA and/or protein is isolated from lung tissue and changes in MUC5 mRNA or protein levels are measured.

In some embodiments, the animal models used for testing comprise induction with ovalbumin, bleomycin, elastase, house dust mite (HDM) models for muco-obstructive or pulmonary fibrosis diseases. In other embodiments, the animal models are genetic mouse models for muco-obstructive or pulmonary fibrosis diseases. In other embodiments, the animal models comprise lipopolysaccharide (LPS)-exposed animals, or a combination thereof. In other embodiments, the animal model of lung disease comprises exposure to cigarette smoke.

### Mucus Measurements

### Molecular methods

In some embodiments, the molecular methods used for measuring mucus comprise methods that are well-known in the art. In some embodiments, the methods comprise RT-PCR, quantitative RT-PCR; Northern-blot (RNA-blot) assay; luciferase reporter and chromatin immunoprecipitation (ChIP) assay (promoter-binding); transgenic or knockout animals; or a combination thereof.

### Protein detection methods

In some embodiments, the protein detection methods used for measuring mucus comprise methods that are well-known in the art. In some embodiments, the methods comprise determination of percent solid matter; ELISA; SDS-PAGE/western blot assay; Dot-blot (Slot-blot) assay, immune-fluorescence; or a combination thereof.

### Biochemical and biophysical methods

In some embodiments, the biochemical and biophysical methods used for measuring mucins comprise methods that are well-known in the art. In some embodiments, the methods comprise isolation, fractionation and purification of mucins; glycosylation analysis and mass spectrometry, measuring viscoelasticity of mucus (laser/light scattering analysis, direct rheometry, and fluorescence recovery after photobleaching (FRAP); or a combination thereof.

### Kits

In some embodiments, the present invention provides a kit comprising a therapeutic or prophylactic composition containing an effective amount of one or more antisense oligonucleotides, as described herein, and optionally a second composition comprising one or more therapeutic or prophylactic agents for treating a disease or disorder as described herein. In certain embodiments, the kit comprises a sterile container which contains therapeutic or prophylactic agents; such containers can be boxes, ampules, bottles, vials, tubes, bags, pouches, blister-packs, or other suitable container forms known in the art. Such containers can be made of plastic, glass, laminated paper, metal foil, or other materials suitable for holding medicaments. In some embodiments, the container is made of a material selected from the group consisting of thin-walled film or plastic (transparent or opaque), paperboard-based, foil, rigid plastic, metal (e.g., aluminum), glass, etc.

If desired, the composition(s) are provided together with instructions for administering the composition(s) to a subject having or at risk of developing a lung disease or disorder. The instructions will generally include information about the use of the composition for the treatment or prevention of a lung disease or disorder. In other embodiments, the instructions include at least one of the following: description of the therapeutic agent; dosage schedule and administration for treatment or prevention of a lung disease or disorder or symptoms thereof; precautions; warnings; indications; counter-indications; overdosage information; adverse reactions; animal pharmacology; clinical studies; and/or references. The instructions may be printed directly on the container (when present), or as a label applied to the container, or as a separate sheet, pamphlet, card, or folder supplied in or with the container.

In other embodiments, the instructions are present as an electronic storage data file present on a suitable computer readable storage medium. In other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded. As with the instructions, this means for obtaining the instructions is recorded on a suitable substrate.

In other embodiments, the present invention further provides a kit for identifying a candidate subject for treatment with a composition comprising one or more antisense oligonucleotides, as described herein, and, optionally, a second composition comprising one or more therapeutic or prophylactic agents, and further comprising an evaluator of the gene function of MUC5AC, MUC5B, or both. In some embodiments, the evaluator comprises a DNA sequencing method, as is known in the art. In other embodiments, the evaluator comprises RNA-seq or another RNA sequencing tool to reveal the presence and quantity of RNA in a biological sample. In other embodiments, other methods of evaluating the quantity of downstream MUC5AC, MUC5B DNA, protein, or RNA may be utilized, as are well known in the art. In some embodiments, instructions for use are included in the kit.

In some embodiments, the present invention provides combined preparations. In some embodiments, "a combined preparation" defines especially a "kit of parts" in the sense that the combination partners as defined above can be dosed independently or by use of different fixed combinations with distinguished amounts of the combination partners i.e., simultaneously, concurrently, separately, or sequentially. In some embodiments, the parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation.

In some embodiments, the components of the kit disclosed above are sterile. In some embodiments, the term "sterile" refers to a state of being free from biological contaminants. Any method of sterilization is applicable and would be apparent to one of ordinary skill in the art.

In some embodiments, the content of the kit is packaged, to allow for storage of the components until they are needed.

In some embodiments, the components of the kit are stored in separate containers within the main kit containment element e.g., box or analogous structure, may or may not be an airtight container, e.g., to further preserve the sterility of some or all of the components of the kit.

### Definitions

The definitions set forth herein take precedence over definitions set forth in any patent, patent application, and/or patent application publication incorporated herein by reference.

Listed below are definitions of various terms used to describe the present invention. These definitions apply to the terms as they are used throughout the specification (unless they are otherwise limited in specific instances) either individually or as part of a larger group.

In some embodiments, the terms "a" and "an" refer to "one or more" of the enumerated components. It will be clear to one of ordinary skill in the art that the use of the singular includes the plural unless specifically stated otherwise. Therefore, the terms "a," "an" and "at least one" are used interchangeably in this application.

In some embodiments, the term "administering" refers to bringing in contact with a compound of the present invention. In some embodiments, the compositions are applied locally. In other embodiments, the compositions are applied systemically. Administration can be accomplished to cells or tissue cultures, or to living organisms, for example humans.

In some embodiments, the terms "administering," "administer," or "administration" refer to deliver one or more compounds or compositions to a subject parenterally, enterally, or topically. Illustrative examples of parenteral administration include, but are not limited to, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion. Illustrative examples of enteral administration include, but are not limited to oral, inhalation, intrapulmonary, intranasal, sublingual, and rectal administration. Illustrative examples of topical administration include, but are not limited to, transdermal and vaginal administration. In particular embodiments, an agent or composition is administered parenterally, optionally by intravenous administration or oral administration to a subject.

In some embodiments, a composition of the present invention comprises a pharmaceutically acceptable composition. In some embodiments, the phrase "pharmaceutically acceptable" is employed herein to refer to those compounds, materials, compositions, combinations, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

In some embodiments, a composition or combination of the present invention is administered in a therapeutically effective amount. In some embodiments, a "therapeutically effective amount" is intended to include an amount of a compound of the present invention alone or an amount of the combination of compounds claimed or an amount of a compound of the present invention in combination with other active ingredients effective to decrease MUC5AC, MUC5B, or both MUC5AC and MUC5B, to reduce mucus production, to reduce mucus level, to treat or inhibit a lung disease or disorder, to treat or inhibit chronic obstructive pulmonary disease, asthma, Primary Ciliary Dyskinesia, Cystic Fibrosis, idiopathic pulmonary fibrosis, bronchiectasis, chronic rhinosinusitis (CRS), diffuse panbronchiolitis (DPB), or lung cancer, or a combination thereof. In some embodiments, a "therapeutically effective amount" of a composition of the invention is that amount of composition which is sufficient to provide a beneficial effect to the subject to which the composition is administered.

In some embodiments, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, i.e., arresting its development; and/or (c) relieving the disease-state, i.e., causing regression of the disease state.

In other embodiments, the terms "treatment" or "treating" of a disease, disorder, or condition encompasses alleviation of at least one symptom of the disease, disorder, or condition. In other embodiments, treatment comprises a reduction in the severity of the disease, disorder, or condition. In other embodiments, treatment comprises inhibition of the progression of the disease, disorder, or condition. In some embodiments, treatment need not mean that the disease, disorder, or condition is completely cured. In some embodiments, to be an effective treatment, a composition as described herein need only reduce the severity of a disease, disorder, or condition, reduce the severity of symptoms associated therewith, or provide improvement to a patient or subject's quality of life.

In other embodiments, "treating" refers to therapeutic treatment and, in other embodiments, prophylactic or preventative measures. In some embodiments, the goal of treating is to prevent or lessen the targeted pathologic condition or disorder as described hereinabove. Thus, in some embodiments, treating may include directly affecting or curing, suppressing, inhibiting, preventing, reducing the severity of, delaying the onset of, reducing symptoms associated with the disease, disorder or condition, or a combination thereof. Thus, in some embodiments, "treating" refers inter alia to delaying progression, expediting remission, inducing remission, augmenting remission, speeding recovery, increasing efficacy of or decreasing resistance to alternative therapeutics, or a combination thereof. In some embodiments, "preventing" refers, inter alia, to delaying the onset of symptoms, preventing relapse to a disease, decreasing the number or frequency of relapse episodes, increasing latency between symptomatic episodes, or a combination thereof. In some embodiments, "suppressing" or "inhibiting", refers inter alia to reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging subject survival, or a combination thereof.

In some embodiments, the term "condition" includes anatomic and physiological deviations from the normal anatomic and physiological states which constitute an impairment of the normal state of the living subject or one of its parts and/or that interrupts or modifies the performance of the bodily functions of the subject.

In some embodiments, the terms "subject" or "individual" or "animal" or "patient" or "mammal," refers to any subject. In one embodiment, the subject is a mammal. In one embodiment, the subject is a human. In other embodiments, the subject is a primate, which in some embodiments, is a non-human primate. In other embodiments, the subject is murine, which in some embodiments is a mouse, and, in other embodiments is a rat. In other embodiments, the subject is canine, feline, bovine, equine, caprine, ovine, porcine, simian, ursine, vulpine, or lupine. In some embodiments, the subject is a chicken or fish.

In some embodiments, the subject is male. In other embodiments, the subject is female.

In some embodiments, the subject is a child. In other embodiments, the subject is an infant. In other embodiments, the subject is an adolescent. In other embodiments, the subject is an adult. In some embodiments, the adult subject is age 50 or older. In other embodiments, the adult subject is age 65 or older. In other embodiments, the adult subject is age 75 or older.

In some embodiments, "antisense compound" or "antisense oligonucleotide" as used herein refers to a compound comprising or consisting of an oligonucleotide at least a portion of which is complementary to a target nucleic acid to which it is capable of hybridizing, resulting in at least one antisense activity.

In some embodiments, "oligonucleotide" as used herein refers to a compound comprising a plurality of linked nucleosides. In certain embodiments, an oligonucleotide comprises one or more unmodified ribonucleosides (RNA) and/or unmodified deoxyribonucleosides (DNA) and/or one or more modified nucleosides.

In some embodiments, "nucleoside" as used herein means a compound comprising a nucleobase moiety and a sugar moiety. Nucleosides include, but are not limited to, naturally occurring nucleosides (as found in DNA and RNA) and modified nucleosides. Nucleosides may be linked to a phosphate moiety.

In some embodiments, "oligonucleoside" as used herein refers to an oligonucleotide in which none of the internucleoside linkages contains a phosphorus atom. As used herein, oligonucleotides include oligonucleosides.

In some embodiments, "complementary" refers to the capacity for precise base pairing between two nucleotides. For example, if a nucleotide at a certain position of an oligonucleotide is capable of hydrogen bonding with a nucleotide at the same position of a target nucleic acid as described herein, then the oligonucleotide and the target nucleic acid are considered to be complementary to each other at that position. An oligonucleotide and target nucleic acid are complementary to each other when a sufficient number of corresponding positions in each molecule are occupied by nucleotides that can hydrogen bond with each other through their bases. In other embodiments, the term "complementary" may be used to indicate a sufficient degree of complementarity or precise base pairing such that stable and specific binding occurs between the oligonucleotide and a target nucleic acid (e.g., between an antisense oligonucleotide and an mRNA). Thus, it should be appreciated 100% complementarity is not required for an oligonucleotide to be consider complementary to a target nucleic acid, provided that a sufficient degree of complementarity or precise base pairing exist to achieve stable and specific binding between the oligonucleotide and target nucleic acid. "Fully complementary" or "100% complementary" means each nucleobase of a first nucleic acid has a complementary nucleobase in a second nucleic acid. In some embodiments, a first nucleic acid is an antisense oligonucleotide and a second nucleic acid is a target nucleic acid.

In some embodiments, a "vector" is includes, *inter alia*, any viral vector, as well as any plasmid, cosmid, phage or binary vector in double or single stranded linear or circular form that may or may not be self transmissible or mobilizable, and that can transform prokaryotic or eukaryotic host either by integration into the cellular genome or exist extrachromosomally (e.g., autonomous replicating plasmid with an origin of replication).

In some embodiments, an "expression cassette" as used herein describes a nucleic acid sequence capable of directing expression of a particular nucleotide sequence in an appropriate host cell, which may include a promoter operably linked to the nucleotide sequence of interest that may be operably linked to termination signals. The coding region usually codes for a functional RNA of interest. The expression cassette including the nucleotide sequence of interest may be chimeric. The expression cassette may also be one that is naturally occurring but has been obtained in a recombinant form useful for heterologous expression. The expression of the nucleotide sequence in the expression cassette may be under the control of a constitutive promoter or of a regulatable promoter that initiates transcription only when the host cell is exposed to some particular stimulus. In the case of a multicellular organism, the promoter can also be specific to a particular tissue or organ or stage of development.

Such expression cassettes can include a transcriptional initiation region linked to a nucleotide sequence of interest. Such an expression cassette is provided with a plurality of restriction sites for insertion of the gene of interest to be under the transcriptional regulation of the regulatory regions. The expression cassette may additionally contain selectable marker genes.

In some embodiments, the compositions as described herein comprise one or more antisense oligonucleotides as described herein. In other embodiments, the compositions as described herein consist of one or more antisense oligonucleotides as described herein. In other embodiments, the compositions as described herein consist essentially of one or more antisense oligonucleotides as described herein.

In the description and claims of the present application, each of the verbs, "comprise", "include" and "have" and conjugates thereof, are used to indicate that the object or objects of the verb are not necessarily a complete listing of components, elements or parts of the subject or subjects of the verb.

As used herein, the term "consists essentially of", or variations such as "consist essentially of" or "consisting essentially of", as used throughout the specification and claims, indicate the inclusion of any recited component or group of components, and the optional inclusion of any recited component or group of integers that do not materially change the basic or novel properties of the specified method, structure, or composition.

It is to be understood that the compositions, combinations and methods of the present invention comprising the elements or steps as described herein may, in other embodiments, consist of those elements or steps, or in other embodiments, consist essentially of those elements or steps. In some embodiments, the term "comprise" refers to the inclusion of the indicated active agents, such as the antisense oligonucleotide, as well as inclusion of other active agents, and pharmaceutically or physiologically acceptable carriers, excipients, emollients, stabilizers, etc., as are known in the pharmaceutical industry. In some embodiments, the term "consisting essentially of" refers to a composition, whose only active ingredients are the indicated active ingredients. However, other compounds may be included which are for stabilizing, preserving, etc. the formulation, but are not involved directly in the therapeutic effect of the indicated active ingredients. In some embodiments, the term "consisting essentially of" may refer to components which facilitate the release of the active ingredient. In some embodiments, the term "consisting" refers to a composition, which contains the active ingredients and a pharmaceutically acceptable carrier or excipient.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about". Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

It is to be understood that the sequence set forth in each SEQ ID NO contained herein is independent of any nucleoside analogues or any modification to a sugar moiety, an internucleoside linkage, or a nucleobase. As such, compounds defined by a SEQ ID NO may comprise, independently, one or more nucleoside analogues or modifications to a sugar moiety, an internucleoside linkage, or a nucleoside. Moreover, the nucleotides T and U are used interchangeably in sequence descriptions.

Other terms as used herein are meant to be defined by their well-known meanings in the art.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments unless the embodiment is inoperative without those elements.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

### EXAMPLES

### Materials and Methods

### Oligonucleotide Synthesis

2MOE modified oligonucleotides on a phosphorothioate backbone were synthesized using methods well known in the art. The oligonucleotides as described in **Table 1** were synthesized and used in subsequent experiments.

### 549 Cell line

A549 lung epithelial cells line, which express MUC5AC and MUC5B endogenously were used to analyze the ability of the antisense oligonucleotides to induce exon skipping and MUC5AC RNA degradation.

### Transfection

A549 cells were plated onto 6-well plates with 180,000 cells per well with 10% FCS DMEM and left overnight prior to transfection. Each antisense oligonucleotide as described herein was transfected into the cells using Lipofecatmine 2000 transfection reagent (Invitrogen) according to the lipofectamine 2000 reagent protocol. In each experiment, the effect of treatment with the ASOs on MUC5AC and MUC5B RNA levels was analyzed in comparison to treatment with a control antisense oligonucleotide.

### RNA Extraction

Twenty-four (24) hr following transfection, total RNA was extracted using RNeasy Mini Kit (QIAGEN). RNA concentration was determined using a nanodrop. Complementary DNA (cDNA) synthesis was performed using the High-Capacity cDNA Reverse Transcription Kit (Applied Biosystems). The cDNA was analyzed by PCR and qPCR.

### Quantitative Detection MUC5AC and MUC5B transcripts (qPCR)

Real-time PCR was performed in QuantStudio 3 Real-Time PCR System using TaqMan^{®} Fast Advanced Master Mix (Applied Biosystems) with TaqMan probes specific for transcripts with or without the exon of interest. The expression level was normalized to the transcript levels of GAPDH. Technical duplicates were analyzed for each sample. Analysis was performed using the double delta Ct analysis.

### PCR

PCR was performed using the Platinum^{™} SuperFi^{™} Master Mix (Invitrogen). PCR products were then separated on an agarose gel for detection of the MUC5AC and MUC5B transcripts. The gels were exposed to UV light for visualization, and the PCR products were recorded.

### Primary bronchial and nasal epithelial cell culture.

HNE and HBE cells were thawed for expansion by growing the cells in PneumaCult^{™}-Ex Plus Medium for one passage. After expansion, cells were seeded on porous filters (Transwell Permeable Supports, 6.5 mm Inserts, 24 well plate, 0.4 µm Polyester Membrane, ref.: 3470, Corning Incorporated). One day later, cells were converted to Air-liquid conditions. After amplification, once the filters stopped leaking from the basal to the apical side, the basal medium was switched to PneumaCult^{™}-ALI Maintenance Medium, and the cells differentiated in air liquid interface culture for 5 weeks. Cells were allowed to accumulate mucus for two weeks before experimentation. Once a week, starting 2 weeks after seeding, mucus was cleaned by incubating the cells with KREBS solution up to 3 hours. HNEs were treated for one or two weeks and HBEs were treated for two weeks with 20µM ASO with and without 1ng/ml or 10ng/ml cytokine treatment from the basal side once in two days during ALI medium exchange. Cytokines were added 2 days prior to ASO administration and every time the ASO was added.

### EXAMPLE 1

**Table 1. Selected oligonucleotides**

| | Target 5'->3' | length | SEQ ID NO: | ASO sequence 5'->3' | SEQ ID NO: |
|---|---|---|---|---|---|
| SPL5AC1 | TGCCTTCCTCCA CAGGGT | 18 | 108 | ACCCUGUGGAGGAA GGCA | 1 |
| SPL5AC2 | TTCCTCCACAGG GTCCTG | 18 | 109 | CAGGACCCUGUGGA GGAA | 2 |
| SPL5AC3 | AGCCAGTCTGGG GTCCTCAT | 20 | 110 | AUGAGGACCCCAGA CUGGCU | 3 |
| SPL5AC4 | AGCCAGTCTGGG GTCCTCATT | 21 | 111 | AAUGAGGACCCCAG ACUGGCU | 4 |
| SPL5AC5 | CCAGTCTGGGGT CCTCAT | 18 | 112 | AUGAGGACCCCAGA CUGG | 5 |
| SPL5AC6 | CAGTCTGGGGTC CTCATT | 18 | 113 | AAUGAGGACCCCAG ACUG | 6 |
| SPL5AC7 | CCAGTCTGGGGT CCTCATT | 19 | 114 | AAUGAGGACCCCAG ACUGG | 7 |
| SPL5AC8 | GCCAGTCTGGGG TCCTCATT | 20 | 115 | AAUGAGGACCCCAG ACUGGC | 8 |
| SPL5AC9 | ACCACGATGACA GCCTGCTGGT | 22 | 116 | ACCAGCAGGCUGUC AUCGUGGU | 9 |
| SPL5AC10 | ATGACAGCCTGC TGGTGA | 18 | 117 | UCACCAGCAGGCUG UCAU | 10 |
| SPL5AC11 | CTGTCCACAGAG AAGTATG | 19 | 118 | CAUACUUCUCUGUG GACAG | 11 |
| SPL5AC12 | CACAGAGAAGTA TGCTCAG | 19 | 119 | CUGAGCAUACUUCU CUGUG | 12 |
| SPL5AC13 | AGAGAAGTATGC TCAGCAC | 19 | 120 | GUGCUGAGCAUACU UCUCU | 13 |
| SPL5AC14 | AAGTATGCTCAG CACTGG | 18 | 121 | CCAGUGCUGAGCAU ACUU | 14 |
| SPL5AC15 | AAGTATGCTCAG CACTGGT | 19 | 122 | ACCAGUGCUGAGCA UACUU | 15 |
| SPL5AC16 | AGTATGCTCAGC ACTGGT | 18 | 123 | ACCAGUGCUGAGCA UACU | 16 |
| SPL5AC17 | GTATGCTCAGCA CTGGTG | 18 | 124 | CACCAGUGCUGAGC AUAC | 17 |
| SPL5AC18 | AGTATGCTCAGC ACTGGTG | 19 | 125 | CACCAGUGCUGAGC AUACU | 18 |
| SPL5AC19 | AAGTATGCTCAG CACTGGTG | 20 | 126 | CACCAGUGCUGAGC AUACUU | 19 |
| SPL5AC20 | GGAACCTACTAC TCGGTAAC | 20 | 127 | GUUACCGAGUAGU AGGUUCC | 20 |
| SPL5AC21 | CCTACTACTCGG TAACATC | 19 | 128 | GAUGUUACCGAGU AGUAGG | 21 |
| SPL5AC22 | ACTACTCGGTAA CATCTGC | 19 | 129 | GCAGAUGUUACCGA GUAGU | 22 |
| SPL5AC23 | TCGGTAACATCT GCCGCCT | 19 | 130 | AGGCGGCAGAUGU UACCGA | 23 |
| SPL5AC24 | AGGTGGAGCCGG CCAGGTACTA | 22 | 131 | UAGUACCUGGCCGG CUCCACCU | 24 |
| SPL5AC25 | CCAGGCCTGCCA TGAAGT | 18 | 132 | ACUUCAUGGCAGGC CUGG | 25 |
| SPL5AC26 | GAGCATCTGCCG TGAGTG | 18 | 133 | CACUCACGGCAGAU GCUC | 26 |
| SPL5AC27 | ATCTGCCGTGAG TGCGAGT | 19 | 134 | ACUCGCACUCACGG CAGAU | 27 |
| SPL5AC28 | AGCATCTGCCGT GAGTGCGA | 20 | 135 | UCGCACUCACGGCA GAUGCU | 28 |
| SPLSB1 | AGCACCTGCAGG AACCGGA | 19 | 136 | UCCGGUUCCUGCAG GUGCU | 29 |
| SPL5B2 | CTACGGGGATGG CCACTT | 18 | 137 | AAGUGGCCAUCCCC GUAG | 30 |
| SPL5B4 | CAGGTTGACTCC ACCAAGT | 19 | 138 | ACUUGGUGGAGUC AACCUG | 31 |
| SPL5B5 | GGTTGACTCCAC CAAGTAC | 19 | 139 | GUACUUGGUGGAG UCAACC | 32 |
| SPL5B6 | ACTGCGAGTGTT TCTGCA | 18 | 140 | UGCAGAAACACUCG CAGU | 33 |
| SPL5B7 | GACTGCGAGTGT TTCTGCA | 19 | 141 | UGCAGAAACACUCG CAGUC | 34 |
| SPL5B8 | ACTGCGAGTGTT TCTGCAC | 19 | 142 | GUGCAGAAACACUC GCAGU | 35 |
| SPL5B9 | GACTGCGAGTGT TTCTGCAC | 20 | 143 | GUGCAGAAACACUC GCAGUC | 36 |
| SPL5B10 | GACTGCGAGTGT TTCTGC | 18 | 144 | GCAGAAACACUCGC AGUC | 37 |
| SPLSB11 | CTGCGAGTGTTT CTGCAC | 18 | 145 | GUGCAGAAACACUC GCAG | 38 |
| SPL5B12 | ACACCTGCCGTG AGTCGG | 18 | 146 | CCGACUCACGGCAG GUGU | 39 |
| SPL5B13 | CCTTCTTCAATG AGGACCA | 19 | 147 | UGGUCCUCAUUGAA GAAGG | 40 |
| SPL5B14 | TTCTTCAATGAG GACCAGATG | 21 | 148 | CAUCUGGUCCUCAU UGAAGAA | 41 |
| SPL5B 15 | CTTCAATGAGGA CCAGATG | 19 | 149 | CAUCUGGUCCUCAU UGAAG | 42 |
| SPL5B16 | TCAATGAGGACC AGATGAAG | 20 | 150 | CUUCAUCUGGUCCU CAUUGA | 43 |
| SPL5B17 | CAATGAGGACCA GATGAAGT | 20 | 151 | ACUUCAUCUGGUCC UCAUUG | 44 |
| SPL5B18 | TGAGGACCAGAT GAAGTG | 18 | 152 | CACUUCAUCUGGUC CUCA | 45 |
| SPL5B19 | CCAGTGTGGCTG CTACGA | 18 | 153 | UCGUAGCAGCCACA CUGG | 46 |
| SPL5B20 | ACTGCCAGAGCT GGTGAG | 18 | 154 | CUCACCAGCUCUGG CAGU | 47 |
| SPL5B21 | GAACTGCCAGAG CTGGTGA | 19 | 155 | UCACCAGCUCUGGC AGUUC | 48 |
| SPL5B22 | GGAGAACTGCCA GAGCTGGT | 20 | 156 | ACCAGCUCUGGCAG UUCUCC | 49 |
| SPL5B23 | CTGCCAGAGCTG GTGAGG | 18 | 157 | CCUCACCAGCUCUG GCAG | 50 |
| SPL5B24 | AGAGCTGGTGAG GGGGTGGGAA | 22 | 158 | UUCCCACCCCCUCA CCAGCUCU | 51 |
| SPL5B25 | TGGTGAGGGGGT GGGAAG | 18 | 159 | CUUCCCACCCCCUC ACCA | 52 |
| SPL5AC5-1 | CTTCAGCCAGTC TGGGGT | 18 | 160 | ACCCCAGACUGGCU GAAG | 53 |
| SPL5AC5-2 | TTCAGCCAGTCT GGGGTCCT | 20 | 161 | AGGACCCCAGACUG GCUGAA | 54 |
| SPL5AC5-3 | CAGCCAGTCTGG GGTCCTCATT | 22 | 162 | AAUGAGGACCCCAG ACUGGCUG | 55 |
| SPL5AC5-4 | CCAGTCTGGGGT CCTCATTCA | 21 | 163 | UGAAUGAGGACCCC AGACUGG | 56 |
| SPL5AC5-5 | AGTCTGGGGTCC TCATTCA | 19 | 164 | UGAAUGAGGACCCC AGACU | 57 |
| SPL5AC5-6 | TCTGGGGTCCTC ATTCAG | 18 | 165 | CUGAAUGAGGACCC CAGA | 58 |
| SPL5AC5-7 | TGGGGTCCTCAT TCAGCAGA | 20 | 166 | UCUGCUGAAUGAG GACCCCA | 59 |
| SPL5AC5-8 | GGTCCTCATTCA GCAGAG | 18 | 167 | CUCUGCUGAAUGAG GACC | 60 |
| SPL5AC5-9 | TCCTCATTCAGC AGAGCAG | 19 | 168 | CUGCUCUGCUGAAU GAGGA | 61 |
| SPL5AC5-10 | TCATTCAGCAGA GCAGCAG | 19 | 169 | CUGCUGCUCUGCUG AAUGA | 62 |
| SPL5AC5-11 | ATTCAGCAGAGC AGCAGCTA | 20 | 170 | UAGCUGCUGCUCUG CUGAAU | 63 |
| SPL5AC5-12 | ATTCAGCAGAGC AGCAGCT | 19 | 171 | AGCUGCUGCUCUGC UGAAU | 64 |
| SPL5AC5-13 | TCAGCAGAGCAG CAGCTA | 18 | 172 | UAGCUGCUGCUCUG CUGA | 65 |
| SPL5AC5-14 | AGCAGAGCAGCA GCTACA | 18 | 173 | UGUAGCUGCUGCUC UGCU | 66 |
| SPL5AC5-15 | AGAGCAGCAGCT ACACCAA | 19 | 174 | UUGGUGUAGCUGC UGCUCU | 67 |
| SPL5AC5-16 | AGCAGCAGCTAC ACCAAG | 18 | 175 | CUUGGUGUAGCUGC UGCU | 68 |
| SPL5AC5-17 | AGCAGCTACACC AAGGTG | 18 | 176 | CACCUUGGUGUAGC UGCU | 69 |
| SPL5AC5-18 | AGCTACACCAAG GTGGAG | 18 | 177 | CUCCACCUUGGUGU AGCU | 70 |
| SPL5AC5-19 | TACACCAAGGTG GAGGCCA | 19 | 178 | UGGCCUCCACCUUG GUGUA | 71 |
| SPL5AC5-20 | AGGCTGGGCCTT GTCCTCAT | 20 | 179 | AUGAGGACAAGGCC CAGCCU | 72 |
| SPL5AC5-21 | TGGGCCTTGTCC TCATGT | 18 | 180 | ACAUGAGGACAAG GCCCA | 73 |
| SPL5AC5-22 | GCCTTGTCCTCA TGTGGAA | 19 | 181 | UUCCACAUGAGGAC AAGGC | 74 |
| SPL5AC5-23 | CTTGTCCTCATGT GGAACC | 19 | 182 | GGUUCCACAUGAGG ACAAG | 75 |
| SPL5AC5-24 | TGTCCTCATGTG GAACCA | 18 | 183 | UGGUUCCACAUGAG GACA | 76 |
| SPL5AC5-25 | TCCTCATGTGGA ACCACG | 18 | 184 | CGUGGUUCCACAUG AGGA | 77 |
| SPL5AC5-26 | CATGTGGAACCA CGATGAC | 19 | 185 | GUCAUCGUGGUUCC ACAUG | 78 |
| SPL5AC5-27 | TGTGGAACCACG ATGACA | 18 | 186 | UGUCAUCGUGGUUC CACA | 79 |
| SPL5AC5-28 | TGGAACCACGAT GACAGC | 18 | 187 | GCUGUCAUCGUGGU UCCA | 80 |
| SPL5AC5-29 | GAACCACGATGA CAGCCT | 18 | 188 | AGGCUGUCAUCGUG GUUC | 81 |
| SPL5AC5-30 | ACCACGATGACA GCCTGCT | 19 | 189 | AGCAGGCUGUCAUC GUGGU | 82 |
| SPL5B5-1 | AGGGAGGAGCTG CCTTACA | 19 | 190 | UGUAAGGCAGCUCC UCCCU | 83 |
| SPL5B5-2 | GGGAGGAGCTGC CTTACA | 18 | 191 | UGUAAGGCAGCUCC UCCC | 84 |
| SPL5B5-3 | GGAGGAGCTGCC TTACAG | 18 | 192 | CUGUAAGGCAGCUC CUCC | 85 |
| SPL5B5-4 | CTGCCTTACAGC CGCACT | 18 | 193 | AGUGCGGCUGUAA GGCAG | 86 |
| SPL5B5-5 | TGCCTTACAGCC GCACTG | 18 | 194 | CAGUGCGGCUGUAA GGCA | 87 |
| SPL5B5-6 | TGGAGCAGAGCG GGGACTACAT | 22 | 195 | AUGUAGUCCCCGCU CUGCUCCA | 88 |
| SPL5B5-7 | AGCAGAGCGGG GACTACAT | 19 | 196 | AUGUAGUCCCCGCU CUGCU | 89 |
| SPL5B5-8 | AGAGCGGGGACT ACATCAA | 19 | 197 | UUGAUGUAGUCCCC GCUCU | 90 |
| SPL5B5-9 | AGCGGGGACTAC ATCAAG | 18 | 198 | CUUGAUGUAGUCCC CGCU | 91 |
| SPL5B5-10 | GGGGACTACATC AAGGTCA | 19 | 199 | UGACCUUGAUGUA GUCCCC | 92 |
| SPL5B5-11 | GACTACATCAAG GTCAGC | 18 | 200 | GCUGACCUUGAUGU AGUC | 93 |
| SPL5B5-12 | TACATCAAGGTC AGCATCC | 19 | 201 | GGAUGCUGACCUUG AUGUA | 94 |
| SPL5B5-13 | ATCAAGGTCAGC ATCCGG | 18 | 202 | CCGGAUGCUGACCU UGAU | 95 |
| SPL5B5-14 | AAGGTCAGCATC CGGCTG | 18 | 203 | CAGCCGGAUGCUGA CCUU | 96 |
| SPL5B5-15 | AGCATCCGGCTG GTGCTGACAT | 22 | 204 | AUGUCAGCACCAGC CGGAUGCU | 97 |
| SPL5B5-16 | ATCCGGCTGGTG CTGACAT | 19 | 205 | AUGUCAGCACCAGC CGGAU | 98 |
| SPL5B5-17 | GGCTGGTGCTGA CATTCCT | 19 | 206 | AGGAAUGUCAGCAC CAGCC | 99 |
| SPL5B5-18 | CTGGTGCTGACA TTCCTGT | 19 | 207 | ACAGGAAUGUCAGC ACCAG | 100 |
| SPL5B5-19 | GTGCTGACATTC CTGTGGA | 19 | 208 | UCCACAGGAAUGUC AGCAC | 101 |
| SPL5B5-20 | CTGACATTCCTG TGGAAC | 18 | 209 | GUUCCACAGGAAUG UCAG | 102 |
| SPL5B5-21 | CATTCCTGTGGA ACGGAGA | 19 | 210 | UCUCCGUUCCACAG GAAUG | 103 |
| SPL5B5-22 | TTCCTGTGGAAC GGAGAG | 18 | 211 | CUCUCCGUUCCACA GGAA | 104 |
| SPL5B5-23 | CTGTGGAACGGA GAGGACA | 19 | 212 | UGUCCUCUCCGUUC CACAG | 105 |
| SPL5B5-24 | TGGAACGGAGAG GACAGT | 18 | 213 | ACUGUCCUCUCCGU UCCA | 106 |
| SPL5B5-25 | AACGGAGAGGA CAGTGCC | 18 | 214 | GGCACUGUCCUCUC CGUU | 107 |

### EXAMPLE 2

### MUC5AC

The ASOs that were selected for testing are mapped to their positions relative to Exons 5, 16, or 26 of MUC5AC in **Figure 1****.** The efficacy of the MUC5AC ASOs was examined by RT-PCR (**Figures 2A-2C**) and by RT-qPCR (**Figure 3**). Data from RT-PCR experiments demonstrated that 5 ASOs targeting Exon 5 (**Figure 2A**) and 2 ASOs targeting Exon 16 (**Figure 2B**) of MUC5AC were effective in significantly reducing the level of MUC5AC full length transcripts. The RT-qPCR data confirmed the high efficacy of the flagged ASOs from the RT-PCR experiments (**Figure 3**). The most efficient ASO are marked with stripes in **Figure 3**. SPL5AC3 was tested in further experiments based on the RT-PCR results. These lead candidates ASOs have no off-target effect on the level of MUC5B transcripts as seen in **Figure 4****.**

A walk on MUC5AC exon 5 was performed to identify additional ASO candidates for the reduction of MUC5B RNA levels. These ASOs are mapped to their positions relative to Exons 5 in **Figure 5****.** RT-PCR experiments provided data demonstrating that at least 6 ASOs significantly reduced the level of MUC5AC full length transcripts (**Figures 6A-6B**).

The effect of lead candidate ASO was further examined in human primary cells. The RT-PCR (**Figure 7A**) and RT-qPCR (**Figure 7B**) data show that SPL5AC4 reduces significantly MUC5AC levels in primary Human Nasal Epithelial Cells (HNEs). The ASO was highly efficient in MUC5AC RNA level reduction also following MUC5AC induction by IL-13 as seen in Figures 7A-B. SPL5AC4 and SPL5AC8 were also highly efficient in reducing MUC5AC levels, with and without" IL-13 induction, in primary Human Bronchial Epithelial Cells (HBEs) (**Figure 8**). These lead candidates ASOs have no off-target effect on the level of MUC5B transcripts in these HBE cells (**Figure 9**).

### EXAMPLE 3

### MUC5B

The ASOs that were selected for testing are mapped to their positions relative to Exons 22, 26, or 28 of MUC5B in **Figure 10****.** The efficacy of the MUC5B ASOs was examined by RT-PCR (Figures 11A-C) and by RT-qPCR (**Figure 12**). Data from RT-PCR experiments demonstrated that one ASO targeting Exon 22 and one ASO targeting Exon 26 of MUC5B were effective in significantly reducing the level of MUC5B full length transcripts. The RT-qPCR data confirmed the high efficacy of the flagged ASOs from the RT-PCR experiments for all ASOs (**Figure 12**). Also, one additional Exon 26-targeting ASO was added as a lead candidate (**Figure 12**, SPL5B8) based on the RT-PCR results. The most efficient ASO are marked with stripes. These lead candidates ASOs have no off-target effect on the level of MUC5B transcripts (**Figure 13**).

A walk on MUC5B exon 5 was performed to identify additional ASO candidates for the reduction of MUC5B RNA levels. These ASOs are mapped to their positions relative to Exons 5 in **Figure 14****.** RT-PCR experiments provided data demonstrating that at least 7 ASOs significantly reduced the level of MUC5B full length transcripts (Figures 15A-B). The RT-qPCR data (**Figure 16**) confirmed the significant reduction of the flagged ASOs from the RT-PCR experiments.

The effect of the SPL5B2 ASO was further examined in primary human Bronchial Epithelial Cells (HBEs). SPL5B2 reduces significantly MUC5B levels in HBEs (**Figure 17**).

### EXAMPLE 3

### Muc5ac and Muc5b RNA expression levels in BALB/c mice

The lungs from four WT BALB/c mice were crushed and homogenized, RNA was extracted, and Muc5ac and Muc5b RNA expression levels were analyzed. RT-PCR and RT-qPCR showed that both Muc5ac (**Figures 18A-18B**) and Muc5b (**Figures 18C-18D**) are expressed in this strain. The expression levels of Muc5ac and Muc5b was similar among individual BALB/c mice (Figures 18A-D).

Intrathecal treatment of BALB/c mice with 10 mg/kg SPL5AC4 twice a week for 2 weeks led to reduction of Muc5ac levels measured using RT-PCR (**Figure 19**).
The following numbered embodiments define various aspects and features of the present invention and correspond to the original claims of the parent PCT application PCT/IL2022/051303 (WO 2023/105527):
1. A synthetic antisense oligonucleotide targeting MUC5AC, MUC5B, or both MUC5AC and MUC5 sequences, wherein said oligonucleotide is fully chemically modified.
2. The oligonucleotide of embodiment 1, wherein the antisense oligonucleotide targets exons 4-8, 10-11, 13, 16-22, 26, 28-29, or 31 of MUC5AC, MUC5B, or both MUC5AC and MUC5B.
3. A synthetic antisense oligonucleotide which targets exons 4-8, 10-11, 13, 16-22, 26, 28-29, or 31 of MUC5AC, MUC5B, or both MUC5AC and MUC5B.
4. The oligonucleotide of any one of embodiments 2-3, wherein the antisense oligonucleotide is complementary to:
   a. one or more sequences in said exon;
   b. one or more portions of an intron adjacent to said exon, wherein said intron is 1-60 nucleotides upstream or downstream of said exon; or
   c. the intron-exon junction of said exon.
5. The oligonucleotide of embodiment 4, wherein said intron is 25-35 nucleotides upstream or downstream of said exon.
6. The oligonucleotide of any one of embodiments 1-5, wherein the oligonucleotide targets MUC5AC.
7. The oligonucleotide of embodiment 6, wherein the oligonucleotide targets a portion of any one or more of SEQ ID NOs: 215-232 or 272 or SEQ ID NOs: 2290-2297 or SEQ ID NOs: 251-260 or 271.
8. The oligonucleotide of any one of embodiments 6-7, wherein the oligonucleotide targets a portion of exon 5, exon 16, or exon 26.
9. The oligonucleotide of embodiment 8, wherein the oligonucleotide comprises any one or more of SEQ ID NOs: 1-25 or 53-82.
10. The oligonucleotide of embodiment 9, wherein the oligonucleotide comprises SEQ ID NOs: 3, 4, 7, 8, 13 or 19, or a combination thereof.
11. The oligonucleotide of embodiment 8, wherein the oligonucleotide comprises SEQ ID NOs: 54, 55, 56, 58, 59, or 80.
12. The oligonucleotide of any one of embodiments 1-5, wherein the oligonucleotide targets MUC5B.
13. The oligonucleotide of embodiment 12, wherein the oligonucleotide targets a portion of any one or more of SEQ ID NOs: 233-250 or 274 or SEQ ID NOs: 2298-2305 or SEQ ID NOs: 261-270 or 273.
14. The oligonucleotide of any one of embodiments 12-13, wherein the oligonucleotide targets a portion of exon 5, exon 22, exon 26, or exon 28.
15. The oligonucleotide of embodiment 14, wherein the oligonucleotide comprises SEQ ID NOs: 29-52 or 83-107.
16. The oligonucleotide of embodiment 13, wherein the oligonucleotide comprises SEQ ID NOs: 32 or 30 or 35.
17. The oligonucleotide of embodiment 14, wherein the oligonucleotide comprises SEQ ID NOs: 88, 91, 94, 95, 101, 104, 105, or 107.
18. The oligonucleotide of any one of embodiments 1-5, wherein the oligonucleotide targets one or more exons of both MUC5AC and MUC5B.
19. The oligonucleotide of embodiment 18, wherein the oligonucleotide targets a portion of exon 4, exon 17, exon 26 of both MUC5AC and MUC5B, or a combination thereof.
20. The oligonucleotide of embodiment 19, wherein the oligonucleotide targets a portion of SEQ ID NO: 251, SEQ ID NO: 233, SEQ ID NO: 224, SEQ ID NO: 242, SEQ ID NO: 230, SEQ ID NO: 248, or a combination thereof.
21. The oligonucleotide of any one of embodiments 1-20, wherein the target sequence comprises a portion of any one or more of SEQ ID NOs: 215-270 or SEQ ID NOs: 2290-2305.
22. The oligonucleotide of any one of embodiments 1-21, wherein said fully chemically modified oligonucleotide comprises a chemically modified backbone.
23. The oligonucleotide of embodiment 22, wherein said chemically modified backbone comprises: a phosphate-ribose backbone, a phosphate-deoxyribose backbone, a phosphorothioate-deoxyribose backbone, a 2'-O-methyl-phosphorothioate backbone, a phosphorodiamidate morpholino backbone, a peptide nucleic acid backbone, a 2-methoxyethyl phosphorothioate backbone, a constrained ethyl backbone, an alternating locked nucleic acid backbone, a phosphorothioate backbone, N3'-P5' phosphoroamidates, 2'-deoxy-2'-fluoro-β-d- arabino nucleic acid, cyclohexene nucleic acid backbone nucleic acid, tricyclo-DNA (tcDNA) nucleic acid backbone, ligand-conjugated antisense, or a combination thereof.
24. The oligonucleotide of embodiment 23, wherein said chemically modified backbone comprises: 2'-O-methoxyethylribose (MOE).
25. The oligonucleotide of any one of embodiments 1-24, wherein said oligonucleotide comprises 14-25 nucleotides.
26. The oligonucleotide of embodiment 25, wherein said oligonucleotide comprises 17-22 nucleotides.
27. A vector comprising one or more of the oligonucleotides of any one of embodiments 1-26.
28. A cell comprising one or more of the oligonucleotides of any one of embodiments 1-26 or the vector of embodiment 27.
29. A pharmaceutical composition comprising one or more of the oligonucleotides of any one of embodiments 1-26.
30. The composition of embodiment 29, wherein said composition is formulated for intrapulmonary administration.
31. The composition of embodiment 29, wherein said composition is formulated for inhalation or intranasal administration.
32. A method for treating a lung disease or disorder in a subject, comprising the step of administering to said subject the oligonucleotide of any one of embodiments 1-26, the vector of embodiment 27, the cell of embodiment 28, or the composition of any one of embodiments 29-31.
33. A method for suppressing or inhibiting a lung disease or disorder in a subject, comprising the step of administering to said subject the oligonucleotide of any one of embodiments 1-26, the vector of embodiment 27, the cell of embodiment 28, or the composition of any one of embodiments 29-31.
34. The method of any one of embodiments 32-33, wherein said lung disease comprises cancer.
35. A method for treating a non-cancerous lung disease or disorder in a subject, comprising the step of administering to said subject a composition comprising a synthetic antisense oligonucleotide targeting MUC5AC, MUC5B, or both MUC5AC and MUC5B sequences.
36. A method for suppressing or inhibiting a non-cancerous lung disease or disorder in a subject, comprising the step of administering to said subject a composition comprising a synthetic antisense oligonucleotide targeting MUC5AC, MUC5B, or both MUC5AC and MUC5B sequences.
37. A method for reducing the levels of MUC5AC, MUC5B, or both MUC5AC and MUC5B in a cell of a subject comprising the step of administering to said subject the oligonucleotide of any one of embodiments 1-26, the vector of embodiment 27, the cell of embodiment 28, or the composition of any one of embodiments 29-31.
38. A method of inducing nonsense-mediated decay of MUC5AC, MUC5B, or both MUC5AC and MUC5B in a cell of a subject comprising the step of administering to said subject a composition comprising a synthetic antisense oligonucleotide targeting MUC5AC, MUC5B, or both MUC5AC and MUC5B sequences.
39. The method of any one of embodiments 32-38, wherein said lung disease or disorder comprises a muco-obstructive disease.
40. The method of embodiment 39, wherein said subject has excess mucus secretion, hyperconcentrated mucus, failed mucus transport, mucus adhesion to airway surfaces, or a combination thereof.
41. The method of any one of embodiments 32-40, wherein said lung disease or disorder comprises chronic obstructive pulmonary disease, asthma, Primary Ciliary Dyskinesia, Cystic Fibrosis, bronchiectasis, or a combination thereof.
42. The method of any one of embodiments 32-41, wherein said lung disease or disorder comprises a pulmonary fibrotic disease.
43. The method of embodiment 42, wherein said pulmonary fibrotic disease comprises idiopathic pulmonary fibrosis (IPF), progressive pulmonary fibrosis (PPF), fibrotic hypersensitivity pneumonitis, connective tissue disease-associated interstitial lung disease (CTD-ILD), or a combination thereof.
44. The method of any one of embodiments 32-43, wherein said subject has lung inflammation.
45. The method of any one of embodiments 32-44, wherein said subject has hyperplasia of goblet cells.
46. The method of any one of embodiments 32-45, wherein said subject has levels of mucus secretion in the normal range.
47. The method of any one of embodiments 32-46, wherein MUC5AC mRNA, MUC5B mRNA, or both MUC5AC and MUC5B mRNA are over-expressed in the airway cells of said subject.
48. The method of any one of embodiments 32-46, wherein said subject has normal expression of MUC5AC, MUC5B, or both MUC5AC and MUC5B mRNA.
49. The method of any one of embodiments 32-46 wherein MUC5AC protein, MUC5B protein, or both MUC5AC and MUC5B protein are over-expressed in the airway cells of said subject.
50. The method of any one of embodiments 32-46, wherein said subject has normal expression of MUC5AC, MUC5B, or both MUC5AC and MUC5B protein.
51. The method of any one of embodiments 32-50, wherein MUC5AC protein, MUC5B protein, or both MUC5AC and MUC5B protein in the airway cells of said subject have increased activation.
52. The method of any one of embodiments 32-50, wherein said subject has normal activation of MUC5AC protein, MUC5B protein, or both MUC5AC and MUC5B protein.
53. The method of any one of embodiments 32-52, wherein the MUC5AC gene, MUC5B gene, or both MUC5AC and MUC5B genes in said subject comprise one or more mutations, polymorphisms, or a combination thereof.
54. The method of embodiment 53, wherein said subject is heterozygous for said mutation or polymorphism.
55. The method of any one of embodiments 53-54, wherein said polymorphism is a single nucleotide polymorphism (SNP).
56. The method of any one of embodiments 32-55, further comprising the step of administering one or more additional treatments to said subject.
57. The method of embodiment 56, wherein said additional treatment comprises a corticosteroid, a short-acting bronchodilator, a long-acting bronchodilator, a combination of methylxanthines and corticosteroids, or any combination thereof.

## Claims

1. A synthetic antisense oligonucleotide targeting MUC5B, wherein said oligonucleotide is fully chemically modified.

2. The oligonucleotide of claim 1, wherein the antisense oligonucleotide targets exons 4-8, 10, 11, 13, 16-22, 26, 28, 29, or 31 of MUC5B.

3. The oligonucleotide of claim 2, wherein the antisense oligonucleotide is complementary to:
a. one or more sequences in said exon;
b. one or more portions of an intron adjacent to said exon, wherein said intron is 1-60 nucleotides upstream or downstream of said exon, for example 25-35 nucleotides upstream or downstream of said exon; or
c. the intron-exon junction of said exon.

4. The oligonucleotide of claim 3, wherein the oligonucleotide targets a portion of any one or more of SEQ ID NOs: 233-250 or 274 or SEQ ID NOs: 2298-2305 or SEQ ID NOs: 261-270 or 273.

5. The oligonucleotide of claim 4, wherein the oligonucleotide targets a portion of exon 5, exon 22, exon 26, or exon 28.

6. The oligonucleotide of claim 5, wherein the oligonucleotide comprises SEQ ID NOs: 29-52 or 83-107, for example SEQ ID NOs: 32, 30, 35, 88, 91, 94, 95, 101, 104, 105, or 107.

7. The oligonucleotide of any of claims 1-6, wherein said fully chemically modified oligonucleotide comprises a chemically modified backbone such as a phosphate-ribose backbone, a phosphate-deoxyribose backbone, a phosphorothioate-deoxyribose backbone, a 2'-O-methyl-phosphorothioate backbone, a phosphorodiamidate morpholino backbone, a peptide nucleic acid backbone, a 2-methoxyethyl phosphorothioate backbone, a constrained ethyl backbone, an alternating locked nucleic acid backbone, a phosphorothioate backbone, N3'-P5' phosphoroamidates, 2'-deoxy-2'-fluoro-β-d-arabino nucleic acid, cyclohexene nucleic acid backbone nucleic acid, tricyclo-DNA (tcDNA) nucleic acid backbone, ligand-conjugated antisense, 2'-O-methoxyethylribose (MOE), or a combination thereof.

8. The oligonucleotide of any of claims 1-7, wherein said oligonucleotide comprises 14-25 nucleotides, for example 17-22 nucleotides.

9. A vector comprising one or more of the oligonucleotides of any of claims 1-8.

10. A cell comprising one or more of the oligonucleotides of any of claims 1-8 or the vector of claim 9.

11. A pharmaceutical composition comprising one or more of the oligonucleotides of any of claims 1-8, the vector of claim 9, or the cell of claim 10, wherein optionally the composition is formulated for intrapulmonary administration, inhalation or intranasal administration.

12. The oligonucleotide of any of claims 1-8, the vector of claim 9, the cell of claim 10, or the composition of claim 11 for use in treating, suppressing or inhibiting a lung disease or disorder in a subject, for use in reducing the levels of MUC5B in a cell of a subject having a lung disease, or for use in inducing nonsense-mediated decay of MUC5B in a cell of a subject having a lung disease.

13. The oligonucleotide, the vector, the cell, or the composition for use according to claim 12, wherein said lung disease comprises cancer, a non-cancerous lung disease, a muco-obstructive disease such as excess mucus secretion, hyperconcentrated mucus, failed mucus transport, mucus adhesion to airway surfaces or levels of mucus secretion in the normal range, chronic obstructive pulmonary disease, asthma, Primary Ciliary Dyskinesia, Cystic Fibrosis, bronchiectasis, a pulmonary fibrotic disease such as idiopathic pulmonary fibrosis (IPF), progressive pulmonary fibrosis (PPF), fibrotic hypersensitivity pneumonitis, interstitial lung disease, connective tissue disease-associated interstitial lung disease (CTD-ILD) such as rheumatoid arthritis (RA)-ILD, lung inflammation, or hyperplasia of goblet cells, or any combination thereof.

14. The oligonucleotide, the vector, the cell, or the composition for use according to any of claims 12-13, wherein MUC5B mRNA, or protein are over-expressed, or wherein MUC5B protein has normal or increased activation, in the airway cells of the subject.

15. The oligonucleotide, the vector, the cell, or the composition for use according to any of claims 12-14, wherein the MUC5B gene in said subject comprises one or more homozygous or heterozygous mutations, polymorphisms, a single nucleotide polymorphism (SNP), or a combination thereof.

16. The oligonucleotide, the vector, the cell, or the composition for use according to any of claims 12-15, wherein one or more additional treatments are administered to the subject, wherein said additional treatments are optionally selected from a corticosteroid, a short-acting bronchodilator, a long-acting bronchodilator, a combination of methylxanthines and corticosteroids, or any combination thereof.
